# EUROPEAN PATENT APPLICATION

(11) **EP 1 676 590 A1**
(43) Date of publication of application: **05.07.2006**
(21) Application number: 04792438.6
(22) Date of filing: 15.10.2004
(51) Int. Cl.: A61K 48/00, A61K 31/409, A61K 33/26, A61K 35/12, A61K 35/76, A61P 35/00, C12N 15/00

(54) **ONCOGENE THERAPEUTIC DRUG**

(30) Priority: 15.10.2003 JP 2003354983
(71) Applicant: The New Industry Research Organization, Kobe-shi, Hyogo 650-0047 (JP)
(72) Inventor: HAMADA, Katsuyuki, Matsuyama-shi, Ehime 7900931 (JP); GOTO, Akinobu, Heathcote Mikage Shironomae, Higashinada-ku, Kobe-shi, Hyogo (JP); SHIRAKAWA, Toshiro, Kobe-shi, Hyogo 6570068 (JP)
(74) Representative: Behnisch, Werner
(86) International application number: PCT/JP2004/015221
(87) International publication number: WO 2005/037322

(57) **Abstract**

A cancer gene therapeutic drug of the present invention includes a carrier cell to be infected with an oncolytic virus, so as to make the oncolytic virus act on a tumor cell within a living body, wherein the carrier cell is selected from A549 cell, 293 cell, SW626 cell and HT-3 cell, showing a potent antitumor effect. Because of use of such cell line having high antitumor effect both *in vitro* and *in vivo* as the carrier cell, the cancer gene therapeutic drug provides more potent antitumor effect in comparison to those of conventional carrier cells.

## Description

### Technical field

This invention relates to a cancer gene therapeutic drug, and a cancer gene therapeutic method using the therapeutic drug.

### Background art

Recently, a cancer gene therapy has been focused attention for cancer therapy and a variety of gene therapies have been proposed and their clinical trials have been carried out. Among them, a clinical trial of a cancer gene therapy using carrier cells was performed by Freeman *et al.,* This cancer gene therapy uses ovarian cancer cells PA-1 with a HSV-tk gene by retrovirus as the carrier cells and its clinical trials for ovarian cancer therapy as well as malignant mesothelioma therapy have been carried out (see non-patent literature Nos. 1 and 2 shown later). Culver *et al.* used mouse NIH-3T3 cells as the carrier cells and performed a clinical trial for cerebral tumor (see non-patent literature No. 3 shown later). Its application for human cancer therapy, however, requires human derived cells as the carrier cells.

A gene therapy using ovarian cancer cells PA-1 as the carrier cells was also carried out by Coukos *et al.* (see non-patent literature No. 4 shown later). This gene therapy constructs an oncolytic virus which specifically proliferates in tumor cells and the virus is infected to the carrier cells (producer cells) and then the infected carrier cells are administered in the tumor site. Herpes simplex 1 (HSV-1) is used as the oncolytic virus. In an animal experiment, their intraperitoneal administration was performed into a nude mouse model with ovarian cancer transferred to peritoneal cavity (see patent literature Nos. 1 and 2 shown later).

Above mentioned ovarian cancer cells PA-1 show high proliferating ability and can be easily manipulated, but they have a drawback of fragility with small cytoplasm. Therefore, introduction of the HSV-tk gene by retrovirus gives little expression of the HSV-tk gene in the tumor site and no satisfactory antitumor effect was obtained against ovarian cancer or malignant mesothelioma.

Application of PA-1 as carrier cells in the cancer gene therapy with the oncolytic virus HSV-1 showed no significant antitumor effect in comparison to that of a therapy with only the oncolytic virus HSV-1. No frequent administrations can be performed in the cancer gene therapy with virus, because of its neutralizing antibody in the blood. Application of PA-1 cells results in little production of virus due to fragile cells. Their disruption before infection to the target tumor cells by cell to cell interaction, and inactivation of the virus with its neutralizing antibody may lead to no significant antitumor effect.

Furthermore, patient's own cancer cells or fibroblasts are used as the carrier cells in a clinical trial of cellular immunological gene therapy. This procedure requires a long time to get a stable cell line and is difficult to manipulate. In addition, inconstant individual difference exists in introduction of gene and it is difficult to get a stable effect.

Non-patent literature No. 1: Human Gene Therapy, **6**, 927-939, 1995
Non-patent literature No. 2: Human Gene Therapy, **9,** 2641-2649, 1998
Non-patent literature No. 3: Science, **256,** 1550-1552, 1992
Non-patent literature No. 4: Clinical Cancer Research, **5,** 1523-1537, 1999
Patent literature No. 1: International Publication No. 99145783, pamphlet
Patent literature No. 2: International Publication No. 01/23004, pamphlet

### Disclosure of the Invention

### Problems to be Solved by the Invention

The purpose of the present invention is to solve the above problems and to find new carrier cells exhibiting potent antitumor effect in the cancer gene therapy using the oncolytic virus, further to establish a new cancer gene therapeutic method exhibiting a very potent antitumor effect using the carrier cells, and to provide a new cancer gene therapeutic drug used for the therapeutic method.

### Means for solving the problems

The inventors of the present invention have investigated for solving the above problems and found those such as (1) more potent antitumor effect can be obtained by using a specific cell line as the carrier cell in comparison to that of conventional carrier cell and (2) induction and raising of a CTL reaction within a living body through prior administration of a virus for immunological treatment, followed by administration of the carrier cell infected with an oncolytic virus, gives a very potent *in vivo* antitumor effect, and accomplished the present invention.

That is, a cancer gene therapeutic drug of the present invention includes a carrier cell to be infected with an oncolytic virus, so as to make the oncolytic virus act on a tumor cell within a living body, wherein the carrier cell is selected from the following (1) to (4) cells:
(1) A549 cell
(2) 293 cell
(3) SW626 cell, and
(4) HT-3 cell (HT-III cell).

As described later, A549 cell is particularly preferable as the carrier cell, among the four kinds of cells.

The oncolytic virus is preferably selected from viruses such as adenovirus, herpesvirus, lentivirus such as HIV virus, retrovirus, reovirus, vesicular stomatitis virus (VSV), and any other oncolytic viruses. Among them, adenovirus gives favorable results as shown later and can be preferably used.

Preferably, the oncolytic virus of the present invention has a tumor specific promoter, according to the kind of cancers to be treated, including such as 1A1.3B promoter (IAI. 3B promoter), midkine (MK) promoter, 13-HCG promoter, SCCA1 promoter, cox-2 promoter, PSA promoter, or any other tumor specific promoters. Any oncolytic virus, capable of infection and proliferation in target tumor cells such as adenovirus including its wild type, can be used in the present invention. Oncolytic viruses without a tumor specific promoter, such as an E1B gene deficient oncolytic adenovirus of ONYX Pharmaceuticals Inc. and an E1A gene partially deficient type Ad5-Δ24 adenovirus of University of Alabama at Birmingham (UAB), may be used.

Preferably,, the present cancer gene therapeutic drug of the present invention may be a drug kit for cancer therapy, which further includes a virus for immunological treatment to be administered for inducing a CTL reaction within the living body to administration of the carrier cell. The virus for immunological treatment is preferably the same kind of virus as the oncolytic virus. The virus for immunological treatment is preferably non-proliferative type and/or inactivated type by UV irradiation etc. Inactivation by UV irradiation etc may shorten the period between administration of the virus for immunological treatment and administration of the carrier cell.

When the cancer gene therapeutic drug of the present invention is a drug kit for cancer therapy, may be the carrier cell, a combination of the virus for immunological treatment and the carrier cell, or further including the oncolytic virus giving a kit composed of three members. Moreover, the kits may include one or more substances of (1)-(4) shown below.
(1) Atelocollagen
(2) GM-CSF (granulocyte-macrophage colony stimulating factor) expression vector to be infected to the carrier cell before administration
(3) Iron preparations
(4) Porphyrin compounds (e.g. 5-aminolevulinic acid: ALA)

Together with the administration of the virus for immunological treatment, or before or after it, administration of an irradiated tumor cell (a patient derived or a generally available one with similar antigen) is preferable for tumor vaccination (tumor immunization). The cancer gene therapeutic drug of the present invention may include such irradiated tumor cell for the tumor vaccination.

Preferable cancer gene therapeutic method using the cancer gene therapeutic drug of the present invention comprises a step for administration of a virus for immunological treatment to induce a CTL reaction within a human body to administration of a carrier cell; and after a predetermined period, a step for at least one administration of a carrier cell to be infected with an oncolytic virus before the administration so as to make the oncolytic virus act on a tumor cell within the human body.

In the above cancer gene therapeutic method, the period from the administration of the virus for immunological treatment to the administration of the carrier cell is preferably set to be two weeks or more, and not more than 13 weeks (more preferably three weeks or four weeks). Preferably, (1) the dose of the virus for immunological treatment may be set between about 10⁵ viral particles and 10¹¹ viral particles for a patient with antibody negative to the virus, while between about 10² viral particles and 10⁷ viral particles for a patient with antibody positive to the virus (for the patient with antibody positive, it may be less than 10² viral particles, or no administrations), (2) the dose of the oncolytic virus through the carrier cell may be set between about 10⁹ viral particles and 10¹⁴ viral particles, (3) the infection rate of the oncolytic virus to the carrier cell may be set between about 0.1 viral particles/cell (hereinafter referred as "vp/cell") and 2,000 vp/cell (more preferably between five vp/cell and 500 vp/cell).

In the above cancer gene therapeutic method, adoption of one or more steps of the following (1) to (5) is preferable:
(1) Administration of the carrier cell by intratumor injection,
(2) Administration of atelocollagen together with the carrier cell,
(3) Administration of the carrier cell having been infected with not only the oncolytic virus, but also a GM-CSF expression vector,
(4) Administration of an iron preparation and/or a porphyrin compound [e.g. 5-aminolevulinic acid (ALA)] together with the carrier cell, and
(5) Administration of a tumor cell for tumor vaccination together with, or before or after the virus for immunological treatment.

### Effect of the Invention

The cancer gene therapeutic drug of the present invention includes, as the carrier cell, a cell line such as A549 cell, which had high antitumor effects both *in vitro* and *in vivo* from the results of screening and therefore, provides more potent antitumor effect in comparison to those of conventional carrier cells.

When the cancer gene therapeutic drug of the present invention is a combination of the two drugs composed of the virus for immunological treatment to be administered in advance and the carrier cell to be administered afterwards, immunological treatment in advance by administration of the virus such as adenovirus and then administration of the carrier cell having been infected with the oncolytic virus provides a direct antitumor effect by infection of the oncolytic virus to target tumor cells, and further induces the CTL reaction within the living body to the infected target cells providing a very potent *in vivo* antitumor effect.

### Brief description of drawings

[Fig. 1] The graph shows the results of proliferation inhibitory effect to ovarian cancer cells HEY using various cell lines as carrier cells, expressed by cell numbers at IC₅₀.
[Fig. 2] The graph shows the results of investigation of proliferation inhibitory effect of oncolytic viruses without and together with carrier cells to ovarian cancer cells HEY in the presence of antiviral antibodies, expressed by antibody titer of anti-adenovirus antibodies at IC₅₀.
[Fig. 3] The graph shows the results of investigation of proliferation inhibitory effect of oncolytic adenovirus infected carrier cells (such as 293 cells) to ovarian cancer cells HEY in the presence of antiviral antibodies, expressed by antibody titer of anti-adenovirus antibodies at IC₅₀.
[Fig. 4] The graph shows the results of investigation of proliferation inhibitory effect to ovarian cancer cells HEY using carrier cells of 293 cells, A549 cells, SW626 cells and HT-3 cells in the presence of antiviral antibodies, expressed by cell numbers.
[Fig. 5] The graph shows the results of investigation of *in vivo* antitumor effect of cancer gene therapeutic drugs of the present invention, using a tumor model with 10-15 mm massive tumor formed by subcutaneous transplantation of human ovarian cancer cells RMG-1 in a nude mouse.
[Fig. 6] The graph shows the results of investigation of *in vivo* antitumor effect of cancer gene therapeutic drugs of the present invention, using a tumor model with 10-15 mm massive tumor formed by subcutaneous transplantation of human ovarian cancer cells PA-1 in a nude mouse.
[Fig. 7] The graph shows the results of investigation of *in vivo* antitumor effect of a cancer gene therapeutic drug of the present invention using a subcutaneouos tumor model mouse [(C57BL/6×C3/Hel) F1 mouse] with normal immune system.
[Fig. 8] The Figure shows a microscopic observation of cell fusion of A549 cells due to administration of adenovirus.
[Fig. 9] The Figure shows a control microscopic observation of A549 cells without administration of adenovirus.
[Fig. 10] Graph (a) shows the results of investigation of midkine (MK) mRNA expression by RT-PCR in human surgical samples of 1-21; graph (b) shows the results of investigation of midkine (MK) mRNA expression by RT-PCR of four cell lines of malignant gliomas in a similar manner; and graph (c) shows the results of investigation of midkine (MK) expression in the above each cell line by Western blotting analysis.
[Fig. 11] The graph shows the results of comparative investigation of a promoter activity in the above each cell line using two different length midkine promoters.
[Fig. 12] Graph (a) shows a schematic structure of the oncolytic adenovirus, having the midkine promoter, designed in the present invention; and graph (b) shows the results of investigation of E1A protein expression in the above each cell line infected with three kinds of adenoviruses by Western blotting analysis.
[Fig. 13] Graph (a) shows the results of comparative investigation of cancer cell proliferation inhibitory effect with three kinds of adenoviruses; graph (b) shows the results of investigation of adenovirus E3 domain's influence on the proliferation inhibitory effect; and graph (c) shows the results of investigation of antitumor effect of adenovirus in a nude mouse subcutaneous transplantation model with tumor of 5 mm diameter.
[Fig. 14] The graph shows the results of antitumor effect of cancer gene therapeutic drugs of the present invention (prepared by infection of oncolytic virus having the midkine promoter to carrier cells) on a massive tumor with 10-15 mm diameter, compared with administration of oncolytic virus without carrier cells.

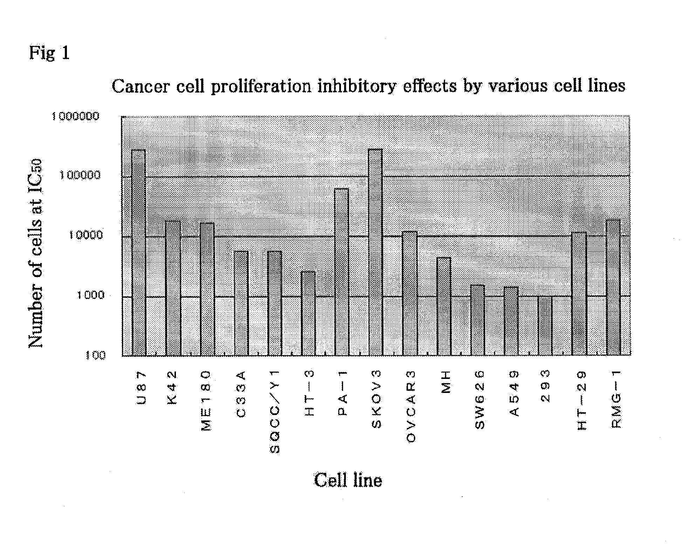

[Fig.1.5] The graph shows the results of investigation of influence of Fe on the proliferation inhibitory effect of adenovirus AdE3-1A1.3B on the ovarian cancer cells PA-1.

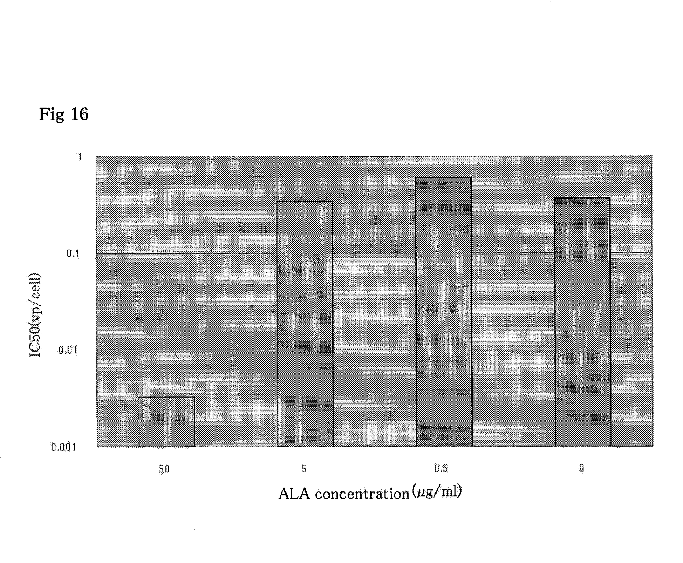

[Fig. 16] The graph shows the results of investigation of influence of ALA on the proliferation inhibitory effect of adenovirus AdE3-1A1.3B on the ovarian cancer cells PA-1.

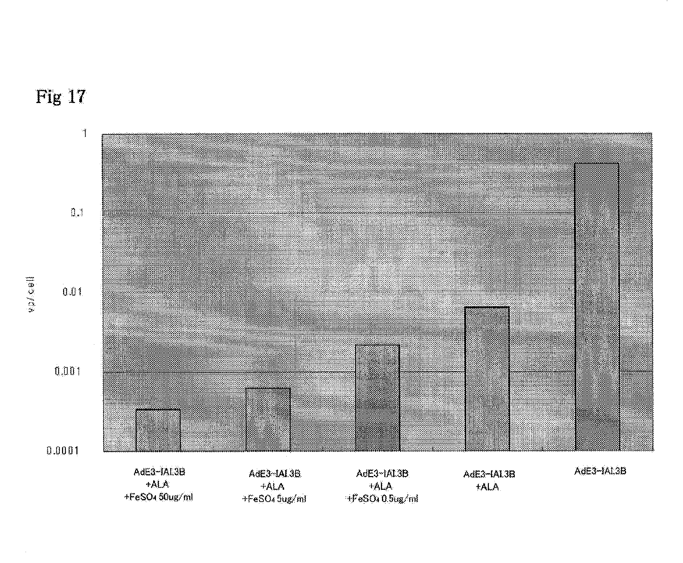

[Fig. 17] The graph shows the results of investigation of influence of the coexistence of Fe and ALA on the proliferation inhibitory effect of adenovirus AdE3-1A1.3B on the ovarian cancer cells PA-1.

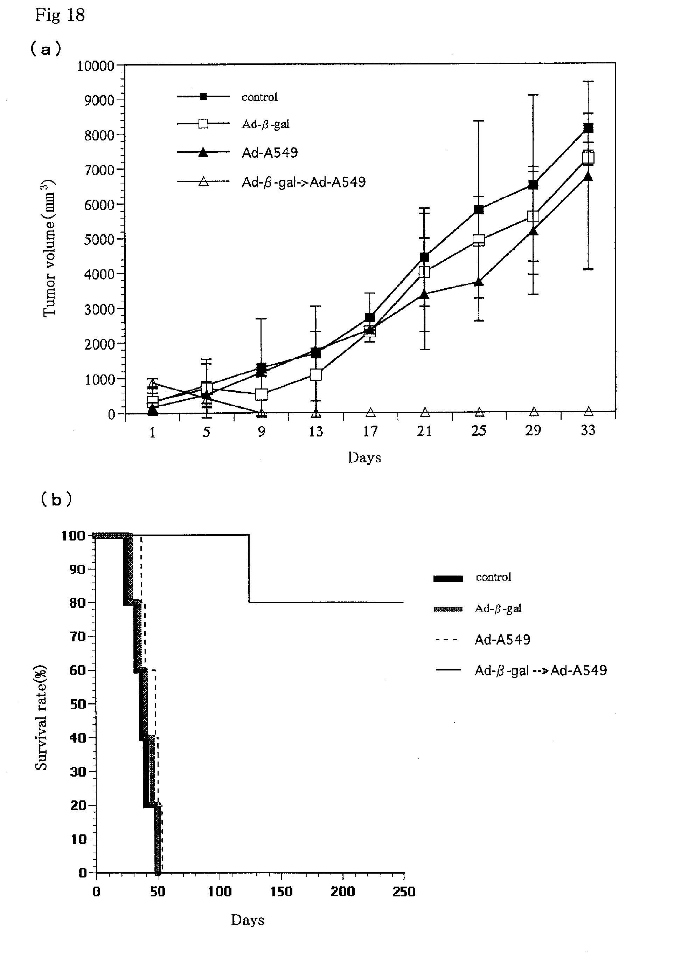

[Fig. 18] The graph (a) shows the results of investigation of *in vivo* antitumor effect of a cancer gene therapeutic drug of the present invention (in the case of no UV irradiation treatment on the virus for immunological treatment) using a subcutaneous tumor model mouse [(C57BL/6×C3/He) F1 mouse] with normal immune system; and graph (b) shows the results of long term observation of survival rate of each mouse used for the experiment.

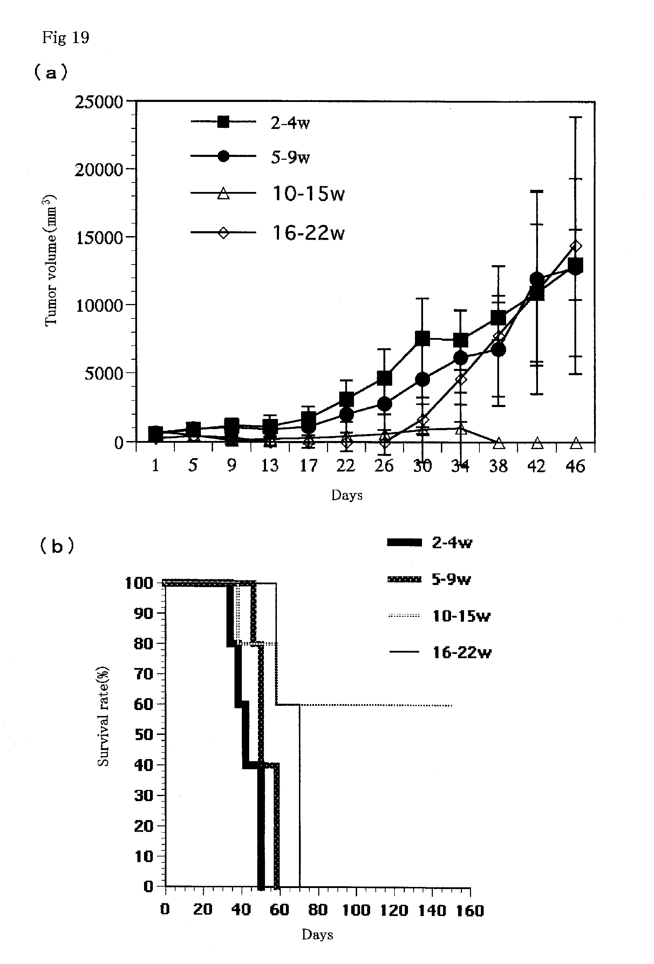

[Fig. 19] The graphs show the results of investigation on the administration interval from the virus for immunological treatment to the carrier cell, graph (a) shows the observed results of tumor volume in each mouse; and graph (b) shows the observed results of the survival rate in each mouse group.

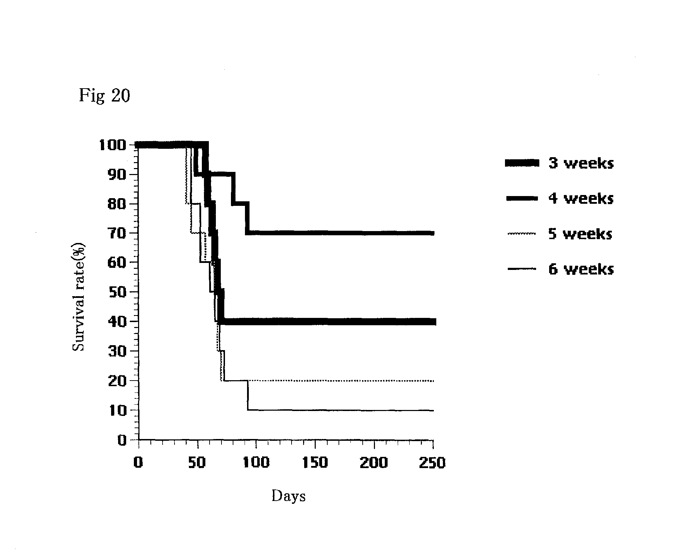

[Fig. 20] The graph (survival curve) shows the results of investigation whether the above administration interval can be shortened by the use of adenovirus UV-Ad-β-gal inactivated by UV irradiation as a virus for immunological treatment.

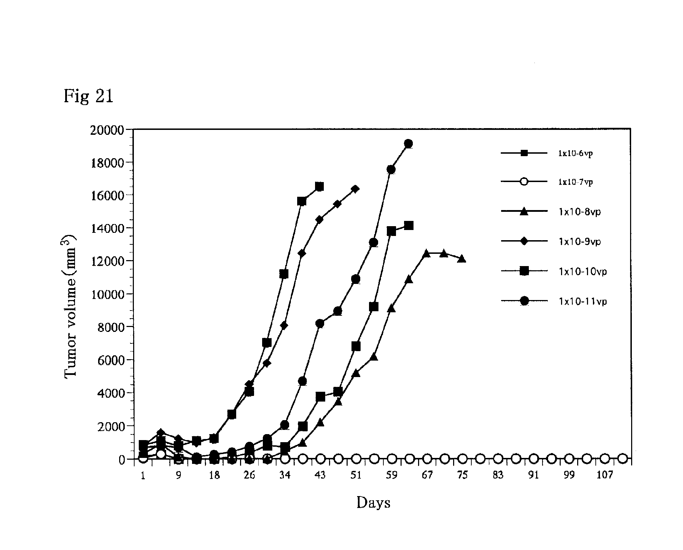

[Fig. 21] The graph (tumor growth curve) shows the results of investigation on the administration rate of the above mentioned UV-Ad-β-gal used as a virus for immunological treatment.

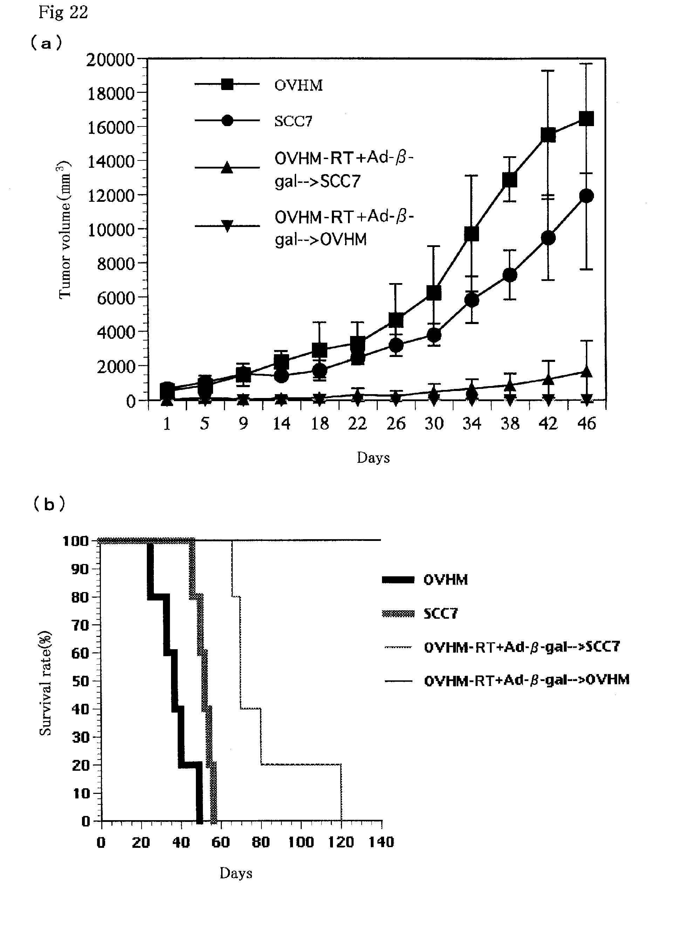

[Fig. 22] The graphs show the examination results of tumor vaccination effect, graph (a) shows the observed results of the tumor volume in each mouse; and graph (b) shows the observed results of survival rate in each mouse. The number (n) of each mouse group including five animals is shown by n = 5. In the Fig., control "SCC7" and "OVHM" show the results of subcutaneous transplantation of squamous epithelium cancer cells SCC7 or ovarian cancer cells OVHM at a rate of 1×10⁶ cells, followed by administration of AdE3-1A1.3B infected carrier cells A549 to mice. "OVHM-RT+ Ad-β-gal→SCC7, OVHM" shows the results of subcutaneous transplantation of SCC7 or OVHM, followed by administration of AdE3-1A1.3B infected carrier cells A549 to mice, after the mice had tumor vaccination with irradiated OVHM and also administration of Ad-β-gal for induction of the CTL to the adenovinis.

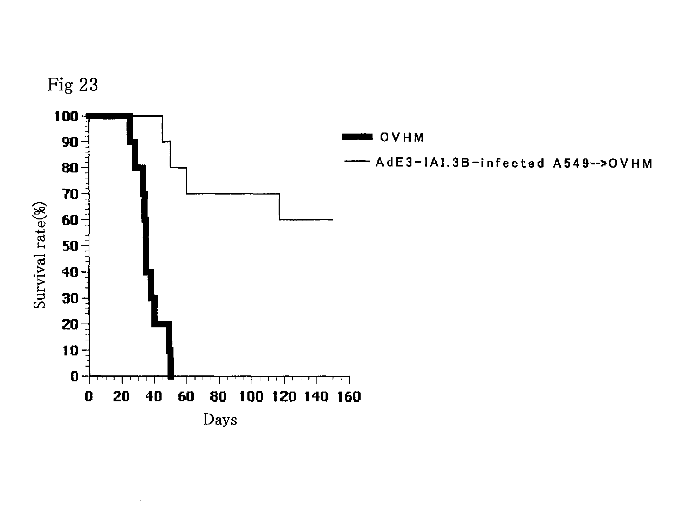

[Fig.23] The graph (survival curve) shows the examination results of tumor vaccination effect with non-small-cell lung cancer A549 cells. The number (n) of each mouse group including 10 animals is shown by n = 10. In the Fig., control "OVHM" shows the results of subcutaneous transplantation of ovarian cancer cells OVHM at a rate of 1×10⁶ cells, followed by administration of AdE3-1A1.3B infected carrier cells A549 to mice without tumor vaccination. "AdE3-1A1.3B-infected A549→OVHM" shows the results of subcutaneous transplantation of ovarian cancer cells OVHM at a rate of 1×10⁶ cells, followed by administration of AdE3-1A1.3B infected carrier cells A549 to mice, after the mice had subcutaneous vaccination with irradiated 1×10⁶ A549 cells infected with AdE3-1A1.3B.

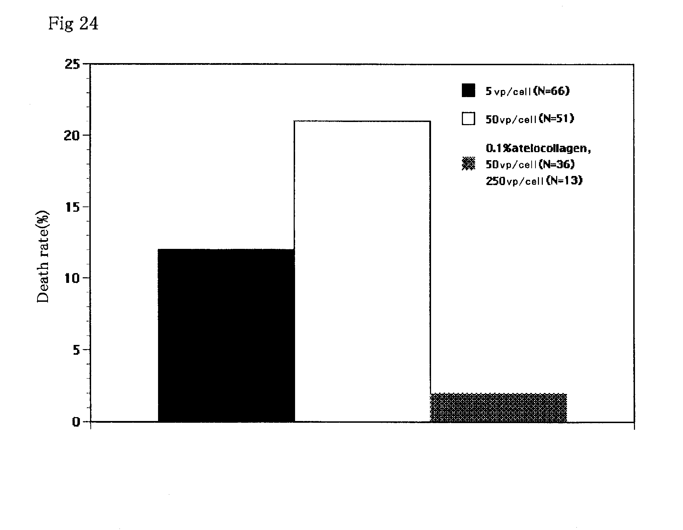

[Fig. 24] The graph shows the results of investigation whether the death rate caused by side effects will be improved by administration of atelocollagen together with the carrier cell. In the Fig., "N" in the parentheses represents number of mouse.

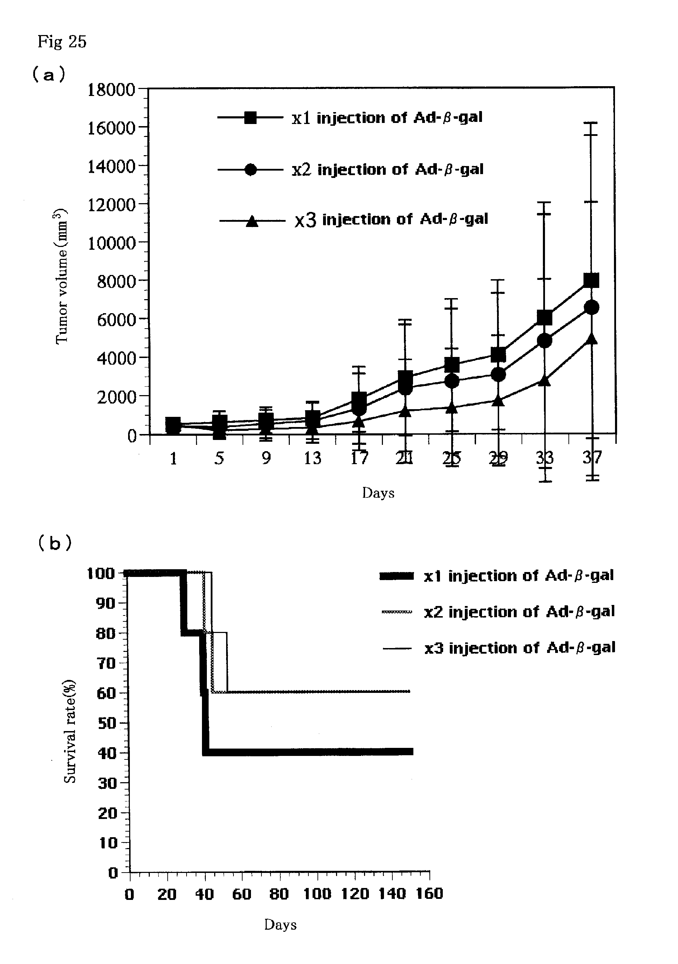

[Fig. 25] The graphs show the results of investigation of antitumor effect in the presence of anti-adenovirus antibodies by 1-3 times administrations of adenovirus Ad-β-gal without UV inactivation treatment, graph (a) shows the observed results of tumor volume of each mouse; and graph (b) shows the observed results of survival rate of each mouse group. A mixture of A549 cells and 293 cells was used as carrier cells. The number (n) of each mouse group including five animals is shown by n = 5.

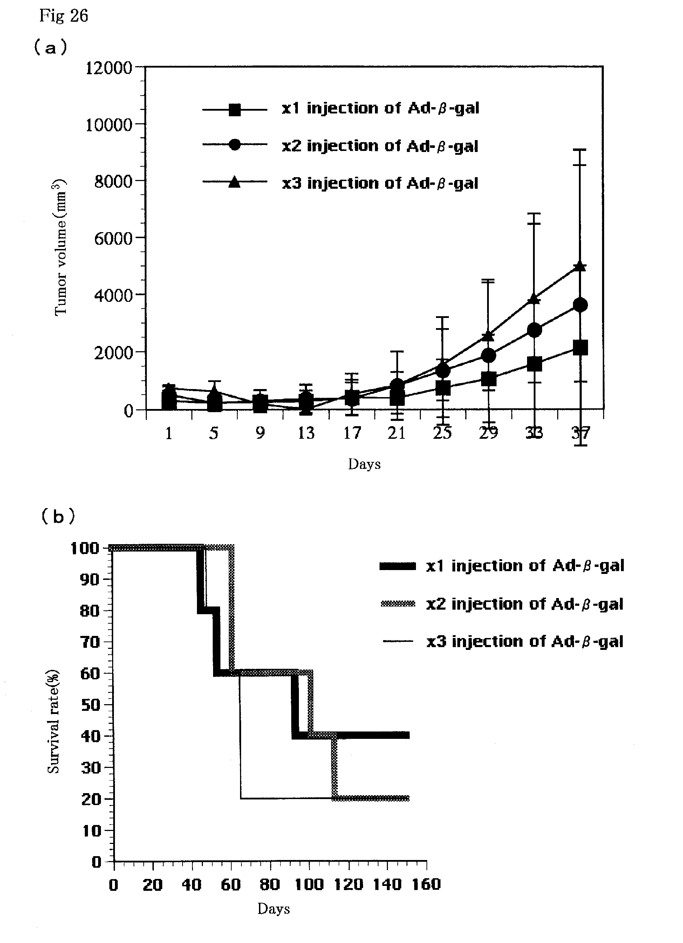

[Fig. 26] The graphs show the results of investigation of antitumor effect in the presence of anti-adenovirus antibodies by 1-3 times administrations of adenovirus Ad-β-gal without UV inactivation treatment, graph (a) shows the observed results of tumor volume of each mouse; and graph (b) shows the observed results of survival rate of each mouse group. A549 cells were used as carrier cells. The number (n) of each mouse group including five animals is shown by n = 5.

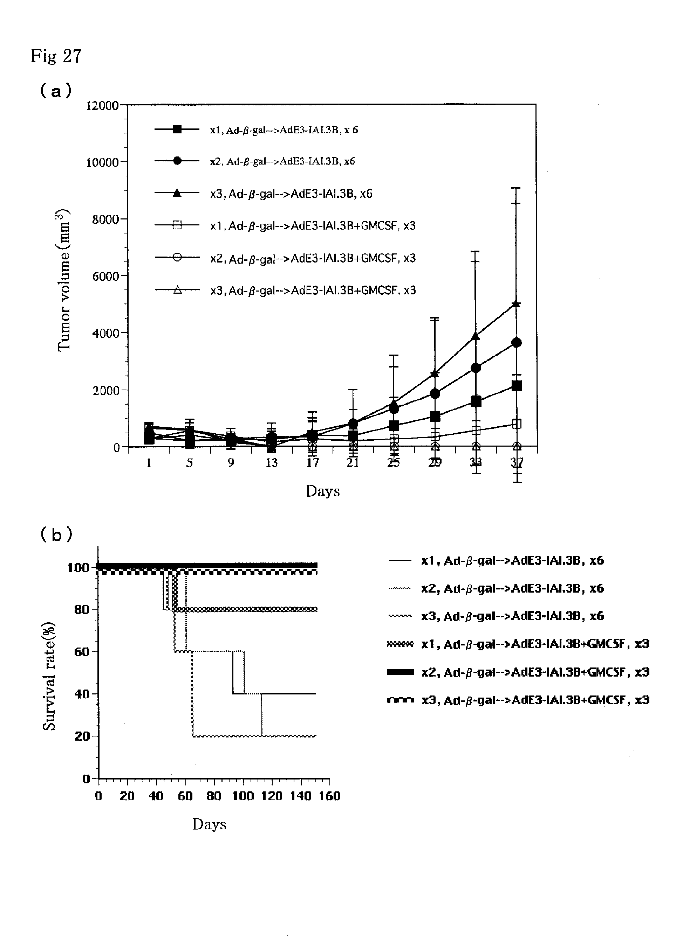

[Fig. 27] The graphs show the results of investigation of *in vivo* antitumor effect of administration of the carrier cells (A549 cells) infected with not only adenovirus AdE3-1A1.3B but also GM-CSF expression vector, and administration of atelocollagen together with the carrier cell, graph (a) shows the observed results of tumor volume of each mouse; and graph (b) shows the observed results of the survival rate of each mouse group. In the Fig., "×1", "×2" and "×3" in front of "Ad-β-gal" represent once, twice and thrice administrations of adenovirus Ad-β-gal, respectively. The number (n) of each mouse group including five animals is shown by n = 5.

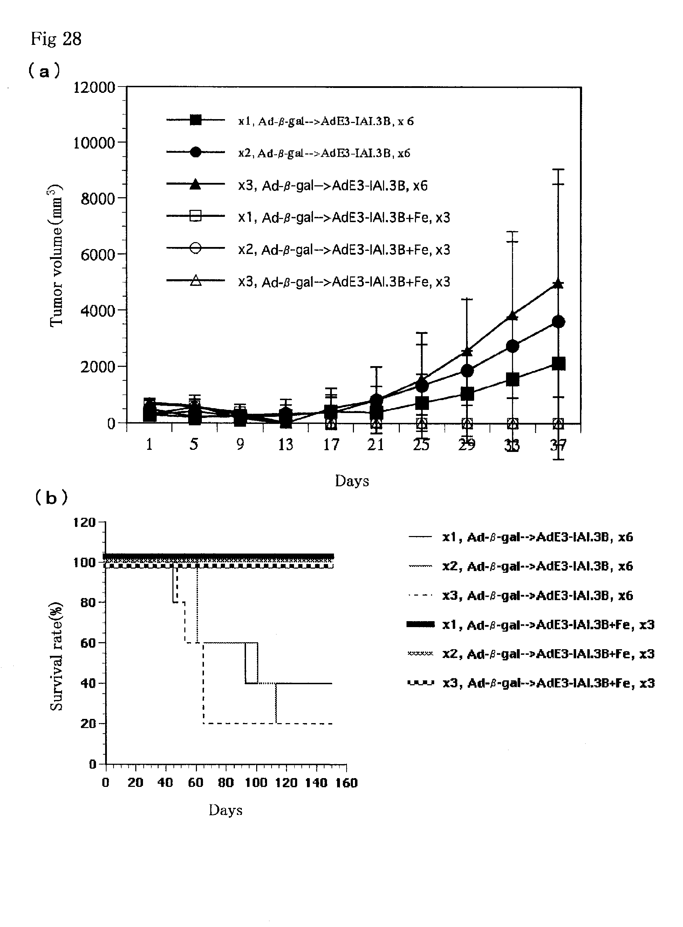

[Fig. 28] The graphs show the results of investigation of *in vivo* antitumor effect of intraperitoneal administration of an iron preparation together with the carrier cell, graph (a) shows the observed results of tumor volume of each mouse; and graph (b) shows the observed results of the survival rate of each mouse group. In the Fig., "×1", "×2" and "×3" in front of "Ad-β-gal" represent once, twice and thrice administrations of adenovirus Ad-β-gal, respectively. The number (n) of each mouse group including five animals is shown by n = 5.

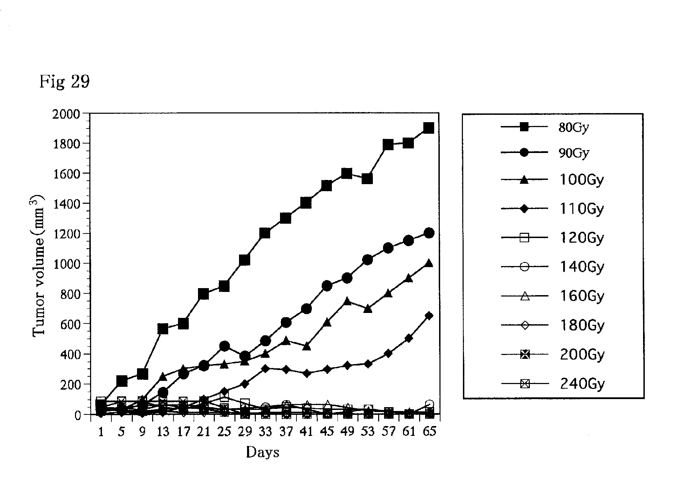

[Fig. 29] The graph shows the results of investigation of radiation dose in radiation exposure to the carrier cell A549 using a nude mouse.

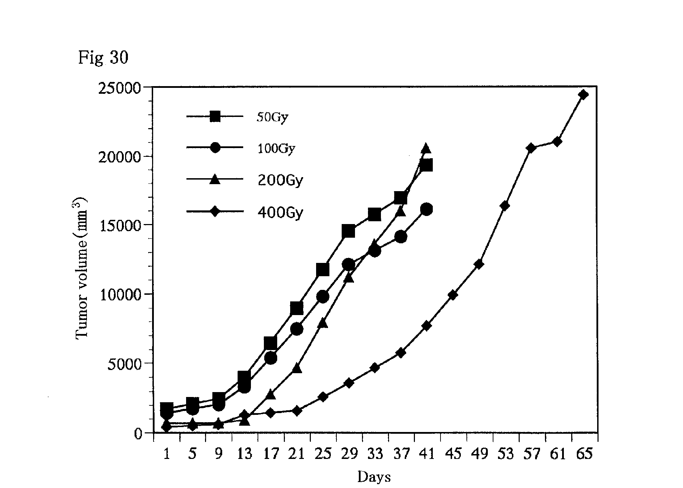

[Fig. 30] The graph shows the results of investigation of antitumor effect of carrier cells A549 irradiated with different doses, using (C57BL/6×C3/He) F1 mouse with subcutaneous transplantation of OVHM.

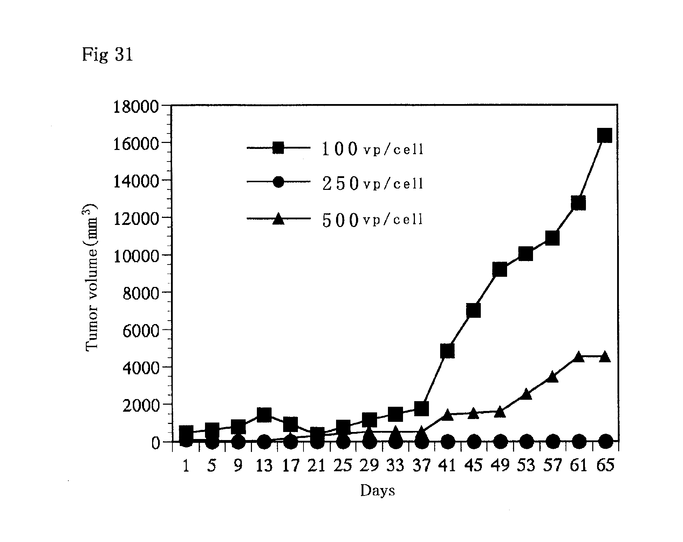

[Fig. 31] The graph shows the results of investigation of the infection rate (amount) of the oncolytic virus to the carrier cell A549.

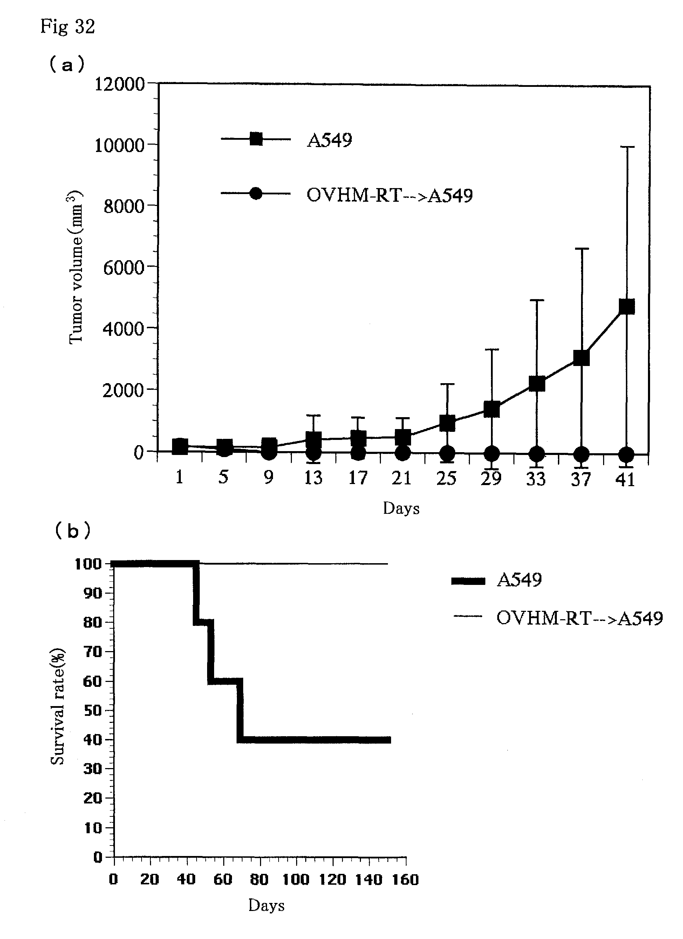

[Fig. 32] The graphs show the examination results of tumor vaccination effect with the ovarian cancer cells OVHM, graph (a) shows the observed results of tumor volume of each mouse; and graph (b) shows the observed results of the survival rate of each mouse group. In the Fig., "A549" shows mouse with three times administrations of AdE3-1A1.3B infected carrier cell A549 without tumor vaccination, "OVHM-RT→A549" shows mouse with three times administrations of AdE3-1A1.3B infected carrier cells A549 after tumor vaccination with irradiated OVHM. The number (n) of each mouse group including five animals is shown by n=5.

### Best mode for carrying out the present invention

One embodiment to carry out the present invention will be explained.
[1] Cancer gene therapeutic drug of the present invention.
A cancer gene therapeutic drug of the present invention includes a carrier cell to be infected with an oncolytic virus, so as to make the oncolytic virus act on a tumor cell within a living body, wherein the carrier cell is selected from the following (1) to (4) cells:
(1) A549 cells
(2) 293 cells
(3) SW626 cells
(4) HT-3 cells (HT-III cells).

Fig. 1 shows the results of screening of the carrier cells to find out effective carrier cells to use for the cancer gene therapeutic drug. More specifically, the cancer gene therapeutic drug was prepared by infection of the oncolytic virus to candidate cell lines and the results of investigation of cancer cell proliferation inhibitory effects are shown. Adenovirus AdE3-1A1.3B (IAI.3B) was used as the oncolytic virus. The adenovirus AdE3-1A1.3B has E1A gene and E3 gene, and an ovarian cancer specific 1A1.3B promoter (IAI.3B promoter) as a tumor specific promoter at the upper stream of E1A gene. The adenovirus AdE3-1A1.3B was infected to various candidate cell lines at a rate of 500 vp/cell for two days and added to the ovarian cancer cells HEY on culture day two, and the proliferation inhibitory effect on the cancer cells HEY was investigated on culture day five.

The vertical axis of Fig. 1 shows cell number capable to obtain 50% proliferation inhibitory effect (IC₅₀) for various candidate cell lines. The less number of cells shows the higher proliferation inhibitory effect. As shown by the Figure, the present investigated cancer cell lines showed high proliferation inhibitory effect in the order of 293 cells, A549 cells, SW626 cells and HT-3 cells (HT- III cells). The 293 cells, A549 cells and SW626 cells exhibited about 100-fold higher proliferation inhibitory effect in comparison to that in PA-1 cells which have been used as carrier cells. HT-3 cells also showed about similar high proliferation inhibitory effect with that of SW626 cells.

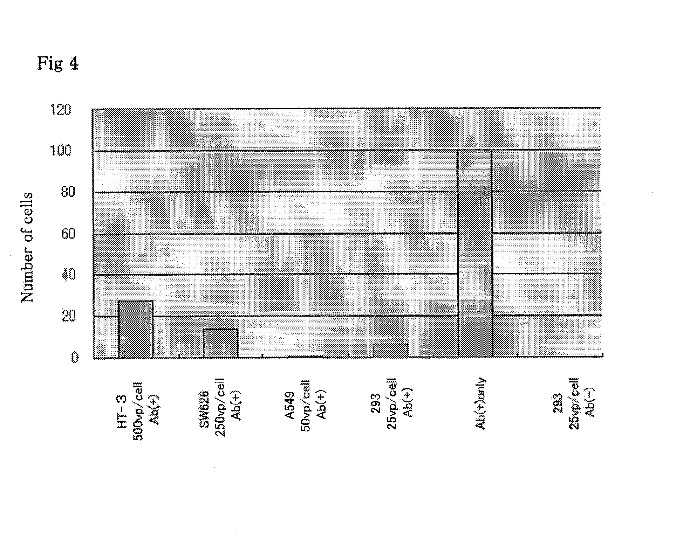

In addition, oncolytic adenovirus was infected to the above mentioned 293 cells, A549 cells, SW626 cells and HT-3 cells to prepare the cancer gene therapeutic drugs and their cancer cell proliferation inhibitory effect was investigated in the presence of a sufficient amount of anti-adenovinis neutralizing antibodies [Ab(+)]. As shown in Fig. 4, all cancer gene therapeutic drugs which used above mentioned four cell lines as carrier cells showed potent cancer cell proliferation inhibitory effect. The conventional cancer gene therapeutic drug with virus was considered to have a difficulty in frequent administrations because of production of antibodies. However, the use of above mentioned four cell lines as carrier cells provided potent *in vitro* proliferation inhibitory effect despite of the presence of antibodies. In addition, A549 cells used as the carrier cell showed most potent proliferation inhibitory effect among above mentioned four cell lines as shown in Fig. 4. That is, administration of the adenovirus infected A549 cells in the presence of a sufficient amount of the anti-adenovirus neutralizing antibodies almost completely inhibited the proliferation of target cancer cells even under the presence of the antibodies.

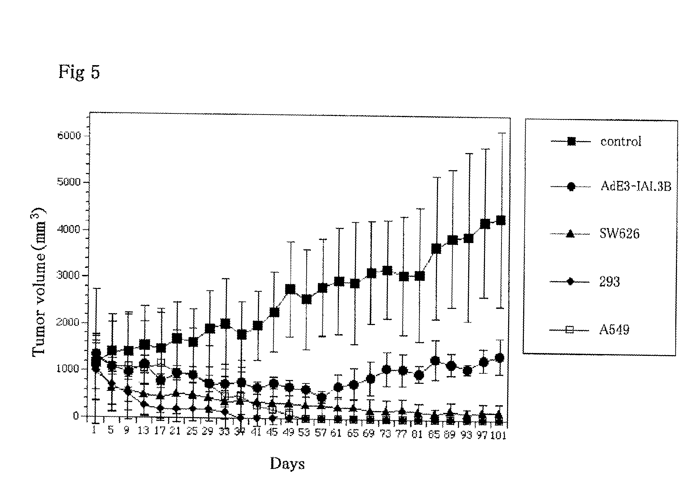

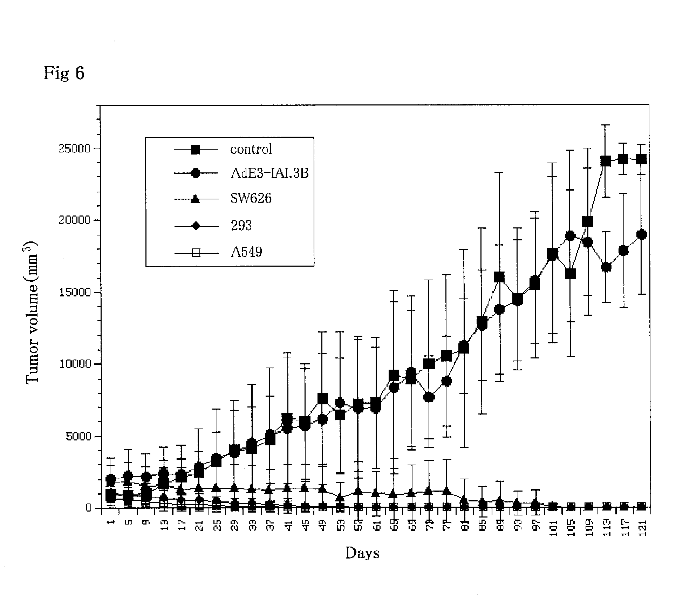

In addition, *in vivo* experiments using a massive subcutaneous tumor nude mouse model of 10-15 mm diameter showed potent antitumor effect when above mentioned A549 cells, 293 cells and SW626 cells were used as the carrier cell (see Fig. 5 and Fig. 6). The details of these experiments will be explained in the examples described later.

As shown above, the cancer gene therapeutic drug obtained by infection of the oncolytic virus to the carrier cell is capable to exhibit high antitumor effect by the use of any one of A549 cells, 293 cells, SW626 cells and HT-3 cells as the carrier cell.

Above mentioned four cell lines are explained. A549 cells are derived from a non-small-cell lung cancer cell line, and their details are described, for example, in the article of Giard, D.J., Aaronson, S.A., Todaro, G.J., Arnstein, P., Kersey, J.H., Dosik, H., and Parks, W.P., *In vitro* cultivation of human tumors: establishment of cell lines derived from a series of solid tumors. J. Natl. Cancer Inst., **51:** 1417-1423, 1923. The 293 cells are derived from human embryonic kidney cells and have been used in many experiments and studies as adenovirus producing cells. The 293 cells are explained, for example, in the article of Xie QW, *et al.,* Complementation analysis of mutants of nitric oxide synthase reveals that the active site requires two hemes. Proc. Natl. Acad. Sci., USA, **93:** 4891-4896, 1996. The SW626 cells are metastatic strain of colon cancer in ovary and their details are described, for example, in the article of Fogh J., *et al.,* Absence of HeLa cell contamination in 169 cell lines derived from human tumors. J. Natl. Cancer Inst., **58:** 209-214, 1977. The HT-3 cells are uterine cervix squamous ep. cancer cells and their details are described, for example, in the article of **Fogh J.,** *et al.,* Absence of HeLa cell contamination in 169 cell lines derived from human tumors. J. Natl. Cancer Inst., **58:** 209-214, 1977. These four cell lines are available from cell preserving organizations such as ATCC (American Type Culture Collection) and other commercially available cells may be used.

A549 cells have many advantages when used as the carrier cell such as (1) production of high titer oncolytic adenovirus and so tough that they can be easily handled, (2) most potent inhibition of proliferation of cancer cells in the presence of anti-adenovirus antibodies, (3) release of secretory granules due to infection of virus, such as adenovirus, because A549 cells are derived from alveolar epithelial cell type II, and the property is favorable in the cancer gene therapy, and (4) resistance to cell elimination effect by CTL even after infection with adenovirus. Therefore, adoption of A549 cells is particularly preferable among above mentioned four cell lines.

Multiple kinds of cells may be used as the carrier cell. A combination of A549 cells and 293 cells in the example described later revealed potent cancer therapeutic effect. Concurrent use of plural kinds of cells may utilize their respective characteristic features and advantages, and it is preferable. For example, SW626 cells require comparatively long period for adhesion and widely disperse in surrounding area by intraperitoneal administration, without restriction in the administered site, and are considered preferable for the intraperitoneal therapy such as ovarian cancer. SW626 cells show characteristic features of late peak in their virus productivity than those of A549 cells and 293 cells, resulting in comparatively longer period of function.

In the cancer gene therapeutic drug of the present invention, a conventional virus vector used for gene introduction may be used as an oncolytic virus to infect the carrier cell. Adenovirus, adeno-accompanying virus, herpes simplex virus type 1 (HSV-1), herpes simplex virus type 2 (HSV-2), *Lentivirus* such as HIV virus (AIDS virus), retroviruses such as mouse leukemia virus, reovirus and vesicular stomatitis virus (VSV) can be exemplified and furthermore other oncolytic viruses may be used. The oncolytic virus is a proliferative virus vector and any virus that modify viral gene so as to specifically proliferate in the target tumor cells or tumor tissues, and fuse or kill target cells with cell lysis (cytolysis) action may be used. For example, adenovirus having E1A or E1B domain necessary for proliferation may be used.

The cancer gene therapeutic drug of the present invention can be applied almost all malignant tumors and may include, for example, ovarian cancer, squamous epithelium cancers (e.g. uterine cervix carcinoma, cutaneous carcinoma, head and neck cancer, esophageal cancer and lung cancer), digestive tract cancers (e.g. colonic cancer, pancreatic cancer, hepatic cancer and gastric cancer), neuroblastoma, cerebral tumor, mammary cancer, testicular cancer and prostatic cancer. In addition, adoption of adenoviruses types 34 and 35 capable of infection to blood cells gives the cancer gene therapeutic drug of the present invention applicable to blood malignant tumors.

Types of the tumor specific promoter to be introduced into the oncolytic virus may be selected according to the kind of target cancer. For example, 1A1.3B promoter for ovarian cancer, midkine promoter for such as cerebral tumor and malignant glioma, β-HCG promoter for testicular cancer, SCCA1 promoter and SCCA2 promoter for squamous epithelium cancers, CEA promoter for colonic cancer, PSA promoter for prostatic cancer and AFP promoter for hepatic cancer may be used. Naturally, other known tumor specific promoters such as cox-2 promoter having a wide action spectrum and exhibiting promoter activity to various malignant tumors and other cancer specific promoters such as osteocarcine promoter may be used. Above mentioned midkine promoter may be used to various malignant tumors in addition to cerebral tumor and malignant glioma and has wide action spectrum as well as cox-2 promoter.

No specific limit is given for the length of each promoter sequence as far as it exhibits the tumor specific promoter activity. Above mentioned 1A1.3B promoter can be designed and prepared according to the disclosures in the pamphlet of International Publication No. 03/025190 and the literature, Cancer Research 63, 2506-2512, 2003 and can be inserted in a virus genome. Above mentioned midlkine promoter, β-HCG promoter and SCCA1 promoter can be designed and prepared according to the disclosures in the pamphlets of International Publication Nos. 02/10368, 01/90344 and 00/60068, respectively.

Above mentioned SCCA1 promoter is explained in detail in the article Biochimica et Biophysica Acta, 91522 (2001) 1-8, Molecular cloning of human squamous cell carcinoma antigen 1 gene and characterization of its promoter, Katsuyuki Hamada, Hiroto Shinomiya, Yoshihiro Asano, Toshimasa Kihana, Mari Iwamoto, Yasushi Hanakawa, Koji Hashimoto, Susumu Hirose, Satoru Kyo and Masaharu Ito.

For example, preparation of an oncolytic adenovirus can be accomplished by insertion of a tumor specific promoter at the upper stream of a primary gene E1A or E1B essential for the proliferation of adenovirus, or replacement with a primary gene E1A or E1B promoter. Similar insertion of the tumor specific promoter at the upper stream of a gene essential for the proliferation of virus is performed when viruses other than adenovirus such as HSV-1, HSV-2, retrovirus, reovirus and vesicular stomatitis virus (VSV) are used for the construction.

However, it is not necessary for the oncolytic virus to have the tumor specific promoter as far as it has specific proliferative property in the target tumor cells or tumor tissues. For example, oncolytic adenoviruses such as an E1B gene deficient type oncolytic adenovirus of ONYX Pharmaceuticals Inc. or an E1A gene partially deficient type Ad5-Δ24 adenovirus of University of Alabama at Birmingham (UAB) may be used. Thus, oncolytic viruses deficient of a tumor specific promoter may be also used. Further, a wild type adenovirus or a partially gene deficient type thereof may be used as the oncolytic virus.

Infection of the oncolytic virus to the carrier cell can be performed by conventional methods without restriction, for example, seeding of carrier cells on a plate, addition of the oncolytic virus at an amount sufficient to infect all cells, cultivation in RPMI medium and fetal calf serum (FCS) (-), under 95% O₂ and 5% CO₂ atmosphere at 37°C for about 6-36 hours, which is simple and easily operable. In the examples shown later, A549 cells, SW626 cells and HT-3 cells were cultured by this method and infected with the oncolytic virus, whereas 293 cells were cultured in DMEM medium and 10% FCS(+) and infected with the oncolytic virus. Fetal calf serum (FCS) is preferably kept under FCS(-) for 3-6 hours infection. Infection for further period is preferably carried out under FCS(-) for 3-6 hours and then FCS is added at a concentration of 10%.

The amount and period of oncolytic virus infection to the carrier cell may be suitably selected according to factors such as the volume and kind of tumor to be treated, kind and rate of the carrier cell, kind of used oncolytic virus and administration method of the cancer gene therapeutic drug of the present invention. Examples are, without particular restriction, for about 6-24 hours at about 5-250 vp/cell by intraperitoneal administration and for about 12-24 hours at about 5-500 vp/cell by intratumoral administration in use of A549 cells, for about 6-24 hours at about 250-2,000 vp/cell by intraperitoneal administration and for about 12-24 hours at about 100-500 vp/cell by intratumoral administration in use of SW626 cells, for about 12-24 hours at about 5-50 vp/cell by intratumoral administration and for about 6-24 hours at about 0.1-10 vp/cell by intraperitoneal administration in use of 293 cells. As shown above, the amount and period of infection vary according to the kinds and administration methods of the carrier cells. The above examples set them within about 6-24 hours at about 0.1-2,000 vp/cell by intraperitoneal administration, and about 12-24 hours at about 5-500 vp/cell by intratumoral administration.

The carrier cell may be kept without infection of the oncolytic virus before use, for the preparation of cancer gene therapeutic drug of the present invention by infection of the oncolytic virus to the carrier cells. Storage of virus infected carrier cells is also preferable in a form prepared by freezing irradiated carrier cells infected with oncolytic virus, and thawing them at the place of medical treatment. The storage of carrier cells may be, for example, performed in a liquid nitrogen or at about -150°C. On the other hand, the oncolytic virus may be kept, for example, at about -80°C.

Before use, the oncolytic virus is infected to the carrier cell by the aforementioned method and the resultant virus infected carrier cells can be administered as it is or together with a conventional pharmaceutical carrier to a human body (or experimental animals such as mouse and rat). As shown later, simultaneous administration of one or more combinations of atelocollagaen, an iron preparation and a porphyrin compound together with the carrier cell is preferable. Administration of carrier cells infected with not only oncolytic virus but also GM-CSF expression vector (virus vector double infected carrier cells) is also preferable.

When the cancer gene therapeutic drug of the present invention is a combination of the carrier cell and the virus for immunological treatment (described later), the carrier cell is administered at a predetermined period after administration of the virus for immunological treatment. When cancer cells are used as the carrier cell, radiation exposure before or after virus infection is preferable. Radiation exposure at a dose of 120-400 Gy, 20-40 Gy or 20-40 Gy was performed before the administration of A459 cells, SW626 cells or HT-3 cells, respectively, as the carrier cell in the examples shown later. The dose of radiation exposure to A549 cells was investigated and no cell proliferation was observed at a dose of 120 Gy or over (Fig. 29) and radiation exposure dose is preferably set between 120 Gy and 600 Gy (more preferably, between 150 Gy and 400 Gy).

The cancer gene therapeutic drug of the present invention may be preferably administered as a parenteral preparation, however, administration as an oral preparation may be also applicable. Administration as a parenteral preparation may be performed by an *in vivo* or *ex vivo* method. The dosage of *in vivo* administration (in other words, the dosage of virus infected carrier cells) may be adjusted according to the volume and kind of tumor, severity of disease, and patient's age and body weight etc. For example, administration may be performed by intracavitary injections such as intravenous injection, intravenous drip infusion, intratumoral injection and intraperitoneal injection. Among them, the carrier cell is preferably administered by intratumoral injection. These injection preparations may be prepared by conventional procedures and general diluents such as a saline solution and a cell culture solution may be used. Furthermore, a bactericide, an antiseptic, a stabilizer, a tonicity agent and an analgesic may be added if necessary. No particular limit is given for the blending quantity of the virus infected carrier cells in these preparations and can be set suitably.

The cancer gene therapeutic drug of the present invention, of course, may be administered in several divided doses to patients or in several divided courses with optional sets of administration times and intervals.

As shown above, the dosage of virus infected carrier cells can be determined according to the volume and kind of tumor, severity of disease, and patient's age and body weight etc. Generally, the dosage of carrier cells can be set between about 10⁷ cells and 10¹⁰ cells for one administration whereas the dosage of oncolytic viruses through the carrier cell can be set between about 10⁹ viral particles and 10¹⁴ viral particles for one administration.

The kind of the carrier cell may be suitably selected according to the kind of cancer to be treated. The carrier cell may be modified by a gene recombinant technology, for example, an artificial expression of a specific protein on the surface of the carrier cell to make easy the binding with the target tumor cells, or treatment such as infection of Sendai virus to the carrier cell.

The oncolytic virus can infect from the carrier cells to the target cells by cell to cell interaction, specifically proliferates in the tumor cells and exerts cell lysis (cytolysis) action of fusion or killing of the tumor cells. The cancer gene therapy with virus was considered to have a difficulty in frequent administrations because of production of its antibodies, however, the carrier cells directly establish infection to the target tumor cells by cell to cell interaction to make frequent administrations possible and a potent antitumor effect can be expected.

[2] The cancer gene therapeutic drug of the present invention, combined with a virus for immunological treatment etc, and its preferred application example
As described below, it is preferable that the cancer gene therapeutic drug of the present invention is combined with a virus for immunological treatment etc.

When the cancer gene therapeutic drug of the present invention is combined with a virus for immunological treatment, it is a combination of: a virus for immunological treatment to be administered for inducing a CTL reaction within a living body to administration of a carrier cell; and a carrier cell having been infected with an oncolytic virus before the administration so as to make the oncolytic virus act on a tumor cell within the living body. In other words, it is a combination of two drugs of a virus for immunological treatment administered in advance and a carrier cell then administered. Administration of the virus for immunological treatment such as adenovirus (immunization in advance) followed by administration of the carrier cell infected with the oncolytic virus induces and raises the CTL reaction within the living body and can obtain a very potent *in vivo* antitumor effect.

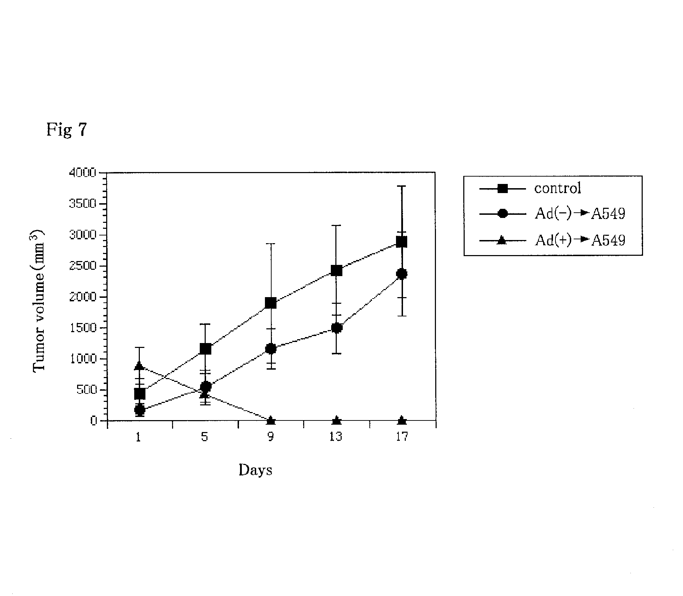

The cancer gene therapeutic drug of the present invention showed a dramatic antitumor effect in a practical experiment using a syngenic model mouse with normal immune system. Although the details will be described later, the ovarian cancer cells OVHM were subcutaneously transplanted to (C57BL/6×C3/He) F1 mouse and were locally injected with carrier cells (A549 cells) infected with oncolytic adenovirus having an ovarian cancer specific promoter. Mice immunized in advance with adenovirus (Ad-β-gal) three months before the injection showed a marked antitumor effect 3-4 days after the start of administration and the tumor was completely disappeared after nine days and lymph node metastasis was also diminished (see Fig. 7 and Fig. 18 and thereafter).

As mentioned above, more potent and dramatic antitumor effect was obtained in the experiment using the mice with normal immune system, producing antibodies. This result shows that the CTL reaction (cytotoxic activity through cytotoxic T lymphocytes) was induced and raised within the living body, by prior administration of the virus for immunological treatment. The conventional cancer gene therapeutic drug with virus was considered to have a difficulty in frequent administrations because of production of its antibodies, however, the cancer gene therapeutic drug of the present invention rather makes use of the immune system within the living body and uses it to attack the virus infected target tumor cells.

The virus for immunological treatment is preferably the same kind as the oncolytic virus. Non-proliferative type and/or inactivated virus may be preferably used as the virus for immunological treatment. Non-proliferative type virus inactivated by treatment such as UV irradiation to disrupt DNA may be more preferably used. For example, the adenovirus with E1 domain deletion and/or inactivated by UV irradiation to disrupt DNA is favorably used as the virus for immunological treatment. Proliferative virus inactivated by UV irradiation etc may be used as the virus for immunological treatment.

In the examples described later, adenovirus (Ad-β-gal) deficient of E1 domain, having LacZ gene encoding β-galactosidase (β-gal) under the control of cytomegalovirus (CMV) promoter, was used for the virus for immunological treatment. Of course, other virus can be used for the virus for immunological treatment. For example, proliferative adenovirus inactivated by UV irradiation may be used. Non-proliferative adenovirus having polyA sequence without integration of any gene (Ad-polyA), inactivated by UV irradiation, may be preferably used.

Dosages of the virus for immunological treatment in the cancer gene therapeutic drug of the present invention are suitably selected according to patient's antibody titer to its virus, volume and kind of tumor, severity of symptoms, and age and body weight of patient. Alteration of dosage in accordance to the antibody positive or negative to its virus is preferable. For example, if adenovirus type 5 is used for the virus for immunological treatment and the oncolytic virus, the rate of the virus for immunological treatment may be set between about 10⁵ viral particles and 10¹¹ viral particles for patients with antibody negative (-), and may be set between about 10² viral particles and 10⁷ viral particles for patients with antibody positive (+) (for patients with antibody positive, it may be less than 10² viral particles, or no administrations). Administration method of the virus for immunological treatment is not restricted, although intracutaneous or subcutaneous injection is preferable.

In addition, the dose of each drug to animals such as mouse and rat may be set at about 1/1,000 to that for a human body in consideration of the differences in body weight, for experimental administration of the cancer gene therapeutic drug of the present invention.

The interval of administration of the virus for immunological treatment and that of the carrier cell may be set between about two weeks and three months. The shorter period is more preferable. In the examples described later, inactivation of the virus for immunological treatment (adenovirus) by UV irradiation shortened the above mentioned interval to about three weeks or four weeks.

Dosages of virus infected carrier cells are as mentioned before and one dose of the oncolytic virus through the carrier cell may be set between about 10⁹ viral particles and 10¹⁴ viral particles, and the infection rate of the oncolytic virus to the carrier cell may be set between about 0.1 vp/cell and 2,000 vp/cell (preferably between 5 vp/cell and 500 vp/cell, more preferably between 150 vp/cell and 400 vp/cell).

For tumor vaccination, administration of tumor cells (cancer cells) together with, before or after the administration of the virus for immunological treatment is preferable. That is, vaccination with the tumor cells (to enhance immunological response of living body to target tumor cells by the administration of the tumor cells treated in advance with radiation exposure, ethanol or formaldehyde) is preferable together with, before or after immunization by the virus for immunological treatment.

Tumor cell derived from patient is preferable for the tumor cells used for the above tumor vaccination (tumor immunization), and commonly available tumor cells with similar antigen may be used. The examples described later investigating the therapeutic effect to ovarian cancer (by OVHM) showed favorable therapeutic effect by use of cancer cells (squamous epithelial cancer cells SCC7 and lung cancer cells A549), for tumor vaccination, different from the target cancer cell to be treated.

In the above tumor vaccination (tumor immunization), no particular limited rate of tumor cell is given. For example, it may be set between about 10⁵ cells and 10¹⁰ cells. The radiation exposure dose to the tumor cells is preferably set between 120 Gy and 600 Gy (more preferably between 200 Gy and 500 Gy). Preferabe administration method is intracutaneous injection or subcutaneous injection.

Furthermore, administration of an iron preparation and/or a porphyrin compound may be used to enhance the viral productivity in the cancer to be treated. Porphyrin compounds such as 5-aminolevulinic acid (ALA), hematoporphyrin and photofirin are exemplified. As iron preparations, ferrous sulfate (FeSO₄ and ferrous citrate for oral administration, and chondroitin sulfate iron and sugar containing iron oxides for intravenous administration may be exemplified. Administration method is not limited, although injection preparation or oral preparation is preferable, together with the cancer gene therapeutic drug of the present invention.

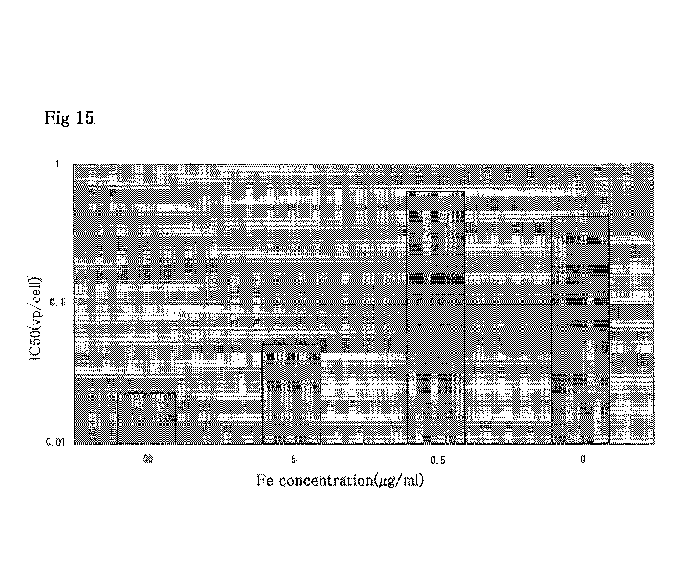

Practically, administration of an iron (Fe) preparation and/or 5-aminolevulinic acid (ALA) could markedly enhance inhibitory effect of the oncolytic adenovirus AdE3-1A1.3B on cancer cell proliferation (see Fig. 15-17 and Fig. 28).

Administration of atelocollagen (product prepared by cleavage of only telopeptide bond of collagen by pepsin treatment etc, making the molecular weight small and water soluble) together with the carrier cell is also preferable. As shown in examples described later, simultaneous administration of atelocollagen and the carrier cell dramatically reduced death rate caused by side effects (Fig. 24). This might be caused by inhibition of dispersion of the oncolytic adenovirus and block against anti-adenovirus antibodies, produced by the atelocollagen.

Therefore, simultaneous administration of atelocollagen and the carrier cell can suppress side effects and realize high dose administration of the oncolytic virus. Commercially available atelocollagen (e.g. a product of Koken Co., Ltd.) or a product prepared by treatment of collagen with pepsin may be used. Atelocollagen is preferably administered by mixing with an injection solution together with carrier cells. Concentration at 0.01-3.0% (w/v) in the solution is considered to exhibit sufficient effect. (Examples described later showed sufficient effect at a low concentration of 0.1-0.2% (w/v) in the solution).

Furthermore, as described earlier, administration of the carrier cells doubly infected with not only oncolytic virus vector but also GM-CSF expression virus vector to enhance the immune response is preferable. (Or, simultaneous administration of two kinds of carrier cells each infected with one of the above virus vectors may be adopted.)

The GM-CSF expression vector is preferable the same kind of virus vector as the oncolytic virus. For example, when adenovirus is used as the oncolytic virus, one may use, for the GM-CSF expression vector, an adenovirus deficient of E1 domain and with a GM-CSF gene encoding granulocyte-macrophage colony stimulating factor (GM-CSF).

In use of the GM-CSF expression vector as well as the oncolytic virus, a total rate of infection of both virus vectors to the carrier cell may be set between five viral particles/cell and 2,000 viral particles/cell.

Application of the GM-CSF expression vector showed highly excellent cancer therapeutic effect (Fig. 27).

Instead of the GM-CSF expression vector, GM-CSF protein may be mixed in an injection solution together with the carrier cells, or the protein's administration by intravenous administration may be taken in consideration.

Methods for use of the cancer gene therapeutic drug of the present invention are, of course, not restricted to the methods described above and various methods for use are available. For example, the cancer gene therapeutic drug of the present invention may be concurrently used with other anticancer agents or a radiation therapy to enhance the infectivity of the oncolytic virus.

A preferred example for use of the cancer gene therapeutic drug of the present invention will be explained by dividing into (1) patients with antibody negative and (2) patients with antibody positive to the virus for immunological treatment.

In the above case (1), non-proliferative adenovirus inactivated by UV irradiation, as described earlier, may be used for the virus for immunological treatment. The amount is about 10⁵ vp to 10¹¹ vp. Patient's derived tumor cells (cancer cells) irradiated at about 200 Gy for tumor vaccination may be administered at 10⁵ cells to 10¹⁰ cells together with the virus for immunological treatment. The virus for immunological treatment and the tumor cell may be administered by intracutaneous or subcutaneous injection.

About 3-4 weeks after administrations of the virus for immunological treatment and the tumor cell, the carrier cell may be administered by intratumoral administration. The dose of the carrier cells may be set about 1×10⁷ to 1×10¹⁰ cells for one administration. A549 cells, irradiated at about 150 Gy to 400 Gy and then administered, may be used as the carrier cell. The adenovirus may be used for the oncolytic virus and the GM-CSF expression vector, and may be infected to the carrier cell at about 250 vp/cell and 5-20 vp/cell, respectively. Atelocollagen may be mixed with an injection solution at a concentration of about 0.1-0.2% and then administered. Simultaneously, an iron (Fe) preparation may be intravenously administered at a dose about 40-100 mg. ALA may be simultaneously administered into the tumor at a dose of 2-2,000 mg.
As mentioned above, the carrier cells etc may be administered at one time. The carrier cells etc may be 1-6 times administered. Administration in plural times may be carried out in consecutive days or at intervals of 2-3 days.

In the above case (2) of patients with positive antibodies, the cancer gene therapeutic drug of the present invention may be administered in a similar manner with that of the above case (1), except for setting the amont of the virus for immunological treatment at about 10⁵ vp or less.

Practical examples of the cancer gene therapeutic drug of the present invention are such as (1) the specific carrier cell, (2) a combination of the carrier cell and the oncolytic virus for the infection to the carrier cell, (3) a combination with the virus for immunological treatment added to the above combinations (1) or (2), (4) a combination with atelocollagen added to the above combinations (1)-(3), (5) a combination with GM-CSF expression vector added to the above combinations (1)-(4), (6) a combination with an iron preparation and/or a porphyrin compound added to the above combinations (1)-(5) to enhance the viral productivity, (7) a combination with the tumor cell for tumor vaccination added to the above combinations (1)-(6), and (8) a combination with necessary compounds for storage, infection and culture, and preparation of medical preparations (e.g. a reagent, a buffer and an enzyme), or vessels (e.g. for reaction, infection and culture, and storage) added to the above combinations (1)-(7).

### Examples

Examples of the present invention are explained referring the drawings, however, the scope of the present invention is not restricted by these examples.

[Example 1: Screening of the carrier cell and antitumor effect in the presence of antibodies]
The following experiments were carried out to screen cancer cell lines which exhibit potent cancer cell proliferation inhibitory effect as the carrier cell.

Adenovirus AdE3-1A1.3B (IAI.3B) was used as the oncolytic virus for the infection to the carrier cell. The adenovirus AdE3-1A1.3B has E1A gene and E3 gene, and an ovarian cancer specific 1A1.3B promoter (1A1.3B promoter) as a tumor specific promoter at the upper stream of E1A gene. The adenovirus AdE3-1A1.3B was infected to various carrier cells at a rate of 500 vp/cell for two days, and then the carrier cells were added to an ovarian cancer cell line HEY on culture day two and their *in vitro* proliferation inhibitory effects were investigated on culture day five.

The results of above experiment are shown in Fig. 1. The vertical axis of the graph shows cell numbers capable to obtain 50% proliferation inhibitory effect (IC₅₀) for each cell line, and the less cell number shows the higher proliferation inhibitory effect. As shown in the Figure, the cancer cell lines investigated in the present experiment showed high antitumor effect in an order of 293 cells, A549 cells, SW626 cells and FIT.3 (HT-III) cells. The 293 cells, A549 cells and SW626 cells exhibited about 100-fold higher proliferation inhibitory effects in comparison to PA-1 cells which have been used as carrier cells. HT-3 cells also exhibited similar high proliferation inhibitory effect to that of SW626 cells.

Then, it was examined how was difference in the proliferation inhibitory effect between only the oncolytic virus and a combination of the oncolytic virus and the carrier cell, in the presence of antiviral antibodies. As the carrier cell, 293 cell was used and above mentioned adenovirus AdE3-1A1.3B was infected for two days. The resultant adenovirus AdE3-1A1.3B infected 293 cells and their supernatant (AdE3-1A1.3B 293 cell+SUPT) were placed in a 12-well plate in the presence of the anti-adenovirus antibodies. In each well, about 50,000 cells of the ovarian cancer cell line HEY had been cultured from the preceding day. The anti-adenovirus antibodies were prepared by dilution of the antibodies with 600-fold antibody titer to various antibody titers. In the case of only the oncolytic virus, the adenovirus AdE3-1A1.3B was administered in the 12-well plate at a rate of 1,000 vp/cell, in the presence of the anti-adenovirus antibodies. At culture day five, the respective proliferation inhibitory effects on cancer cells (HEY cells) were investigated.

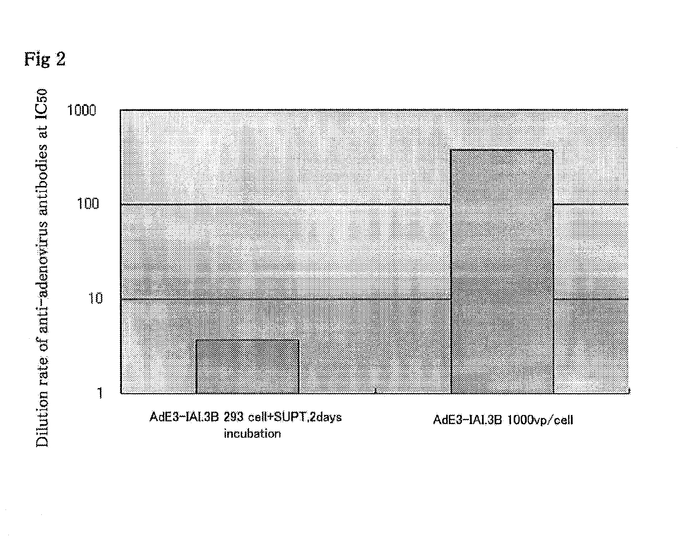

The results of the above experiment are shown in Fig. 2. The vertical axis of the graph shows the dilution rate of the anti-adenovirus antibodies at 50% proliferation inhibitory effect (IC₅₀). In other words, 50% proliferation inhibitory effect was obtained for 293 cells even at about 5-fold dilution rate (120-fold antibody titer) whereas 50% proliferation inhibitory effect was obtained for only the adenovirus at about 600-fold dilution rate (1-fold antibody titer). As shown above, the carrier cell exhibited the proliferation inhibitory effect even under the condition of high antibody titer.

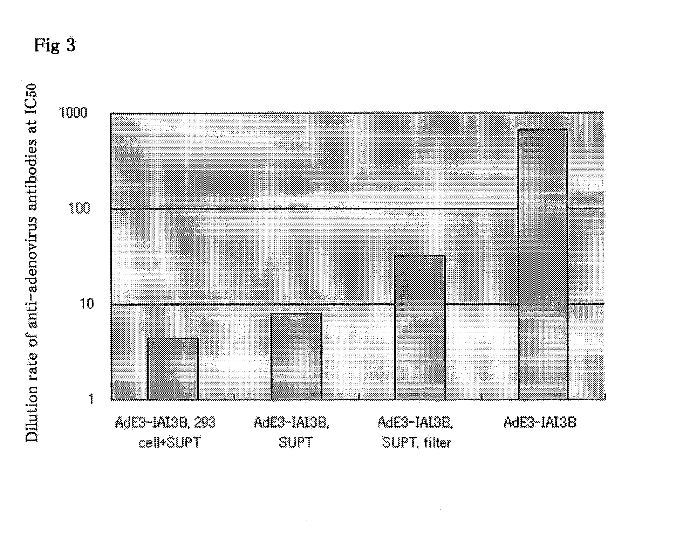

Similarly, the proliferation inhibitory effect to HEY cells was investigated in the presence of anti-adenovirus antibodies in the following conditions, (1) adenovirus infected 293 cell and its supernatant (AdE3-1A13B 293 cell+SUPT), (2) a cell supernatant containing adenovirus (AdE3-1A13B, SUPT), (3) a filtered one with a filter of 0.2µm of the cell supernatant containing adenovirus (AdE3-1A13B, SUPT, filter) and (4) omly the adenovirus (AdE3-1A13B). The results are shown in Fig. 3. The vertical axis of the graph shows a dilution rate of the anti-adenovirus antibodies at 50% proliferation inhibitory rate (IC₅₀). As shown in the Fig., more potent antitumor effect was obtained in comparison to the other conditions when the carrier cell (293 cell) was used.

About carrier cells of 293 cells, A549 cells, SW626 cells and HT-3 cells, each proliferation inhibitory effect on the cancer cells HEY was investigated in the presence [Ab(+)] or absence [Ab(-)] of the anti-adenovirus antibodies having 600-fold antibody titer. The results are shown in Fig. 4. The vertical axis of the graph shows the number of the cancer cells on culture day five. As shown in the Figure, the most potent proliferation inhibitory effect was obtained when A549 cells were used as carrier cells in four kinds of cells. That is, administration of adenovinis infected A549 cells in the presence of a sufficient amount of anti-adenovirus neutralizing antibodies almost completely inhibited the proliferation of the target cancer cells despite of the presence of the antibodies. Other three kinds of cells also showed sufficient proliferation inhibitory effect in the presence of the antibodies.

The cancer gene therapy with virus was considered to have a difficulty in frequent administrations because of production of neutralizing antibodies to the virus. However, application of the carrier cell established direct infection to the target cancer cells by cell to cell interaction and frequent administrations became available. Furthermore, application of the above mentioned four kinds of cells as the carrier cell provided potent antitumor effect.

[Example 2: *In vivo* antitumor effect in a nude mouse subcutaneous tumor model] Then, *in vivo* antitumor effect of the cancer gene therapeutic drug of the present invention was investigated using a nude mouse subcutaneous tumor model. In the experiment, human ovarian cancer cells RMG-1 were subcutaneously transplanted to 5-week-old nude mouse. After four weeks, each carrier cell was injected six times into a massive tumor of about 10-15 mm diameter and the change of the tumor volume was observed. The results are graphically shown in Fig. 5. In the graph, black square represents "control" which are the results of six times injection of PBS buffer into the tumor, black round represents "AdE3-1A1.3B" which are the results of administration of 1×10¹⁰ viral particles per mouse of the adenovirus AdE3-1A1.3B, black triangle shows the results of administration of 1×10⁷ SW626 cells per mouse, infected with the adenovirus AdE3-1A1.3B at 250 vp/cell. Black rhombus shows the results of administration of 1×10⁷ 293 cells per mouse, infected with the adenovirus AdE3-1A1.3B at 25 vp/cell. White square shows the results of administration of 1×10⁷ A549 cells per mouse, infected with the adenovirus AdE3-1A1.3B at 50 vp/cell. As shown in the Figure, 293 cells and A549 cells used as carrier cells showed complete disappearance of the massive tumor with about 10-15 mm diameter 50 days after the administration. SW626 cells showed 98% proliferation inhibitory effect.

Similar experiment as shown above was carried out by subcutaneous transplantation of human ovarian cancer cells PA-1 to 5-week-old nude mouse. The results are shown in Fig. 6. As shown in the Fig., the massive tumor with about 10-15 mm diameter completely disappeared by the use of 293 cells and A549 cells as carrier cells. SW626 cells showed complete disappearance of the tumor in four out of five mice.

[Example 3: *In vivo* antitumor effect in subcutaneous tumor model mouse with normal immune system]
Then, *in* vivo antitumor effect of the cancer gene therapeutic drug of the present invention was investigated using (C57BL/6×C3/He) F1. mouse with normal immune function. In the experiment, each antitumor effect was investigated in the following conditions; (1) the ovarian cancer cells OVHM were subcutaneously transplanted to the syngenic model mouse. After 10 days or more, A549 cells infected with the adenovirus AdE3-1.3B at a rate of 250 vp/cell followed by radiation exposure were six times administered into a formed 5-10 mm tumor, (2) a 7-week-old syngenic model mouse was immunized in advance with an adenovirus for immunological treatment. After three months, the ovarian cancer cells OVHM were subcutaneously transplanted in a similar manner with that of (1) and then, after 10 days or more, A549 cells infected with the adenovirus AdE3-1A1.3B at a rate of 250 vp/cell followed by radiation exposure were six times administered into a tumor, and (3) six times administrations of PBS buffer into a tumor as a control.

The results of the above experiment are shown in a graph of Fig. 7. In the graph, black square represents "control" which are the results of the above condition (3), black round represents "AD(-)→A549" which are the results of the above condition (1), without administration of the adenovirus for immunological treatment. Black triangle represents "Ad(+)→A549" which are the results of the above condition (2), with administration of the adenovirus for immunological treatment. Non-proliferative type adenovirus having no E1 gene was used for the adenovirus for immunological treatment. More specifically, it was an adenovirus with inserted LacZ gene in the downstream of CMV promoter. As shown by the Fig., the above condition (1), without prior immunization by adenovirus, showed 20% antitumor effect in comparison to the control, while the above condition (2), with prior immunization by adenovirus, showed marked antitumor effect 3-4 days after the start of administration and the tumor was completely diminished after nine days with disappearance of lymph node metastasis. As shown by the example, the potent and dramatic antitumor effect in mice with normal immune system, despite of their antibody production, might be caused by induction and raising of the CTL reaction within the living body due to the administration of the adenovirus for immunological treatment.

The oncolytic adenovirus is infected from the carrier cells to the target tumor cells by cell to cell interaction, specifically proliferates in tumor cells and is considered to exert cell lysis (cytolysis) action to fuse and/or kill the target tumor cells. The cancer gene therapeutic drug of the present invention is considered to induce potent CTL reaction within the living body by prior administration of the adenovirus for immunological treatment, which eliminates the oncolytic adenovirus infected target tumor cells and induces complete natural elimination of the adenovirus infected tumor cells.

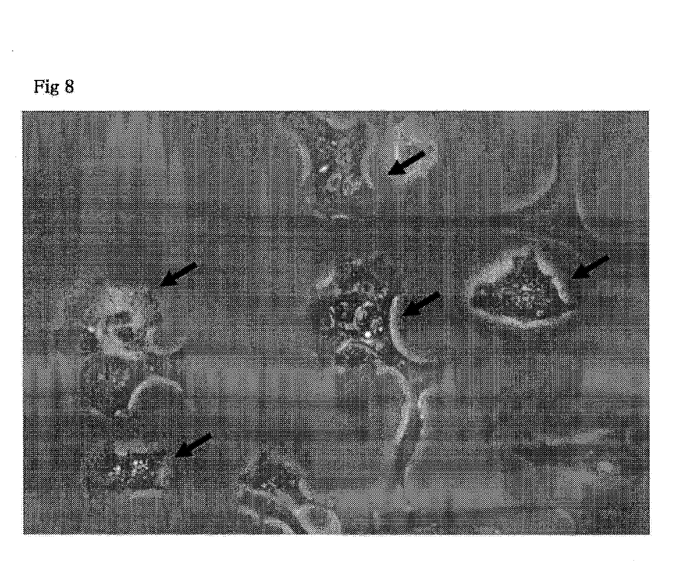

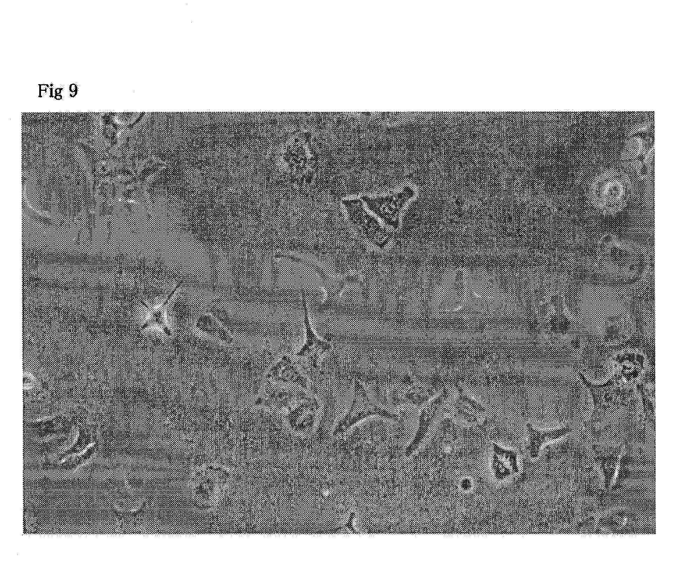

One manner of infection of the adenovirus to the target tumor cell is considered a cell fusion caused by the adenovirus. Fig. 8 shows the result of microscopic observation after overnight culture from the administration of 10,000 viral particles per cell of the adenovirus inactivated by UV irradiation into a well with A549 cells cultured. As shown by arrow marks in the Fig., administration of the adenovirus caused cell fusion and multinucleated cells were sporadically observed. No such cells were observed for A549 cells without adenovirus administration (see Fig. 9).

Predicted infection manners other than cell fusion are a cell adhesion to the target cells of carrier cells, and infection of the adenovirus to the target tumor cells by local burst and the carrier cell fragment including the adenovirus. In any way, proliferation of adenovirus having a tumor specific promoter in the adenovirus infected target tumor cells may lead to presentation of a potent antigen (or, a cancer specific peptide recognized as an antigen secondarily), and the tumor cells may be eliminated by the CTL reaction.

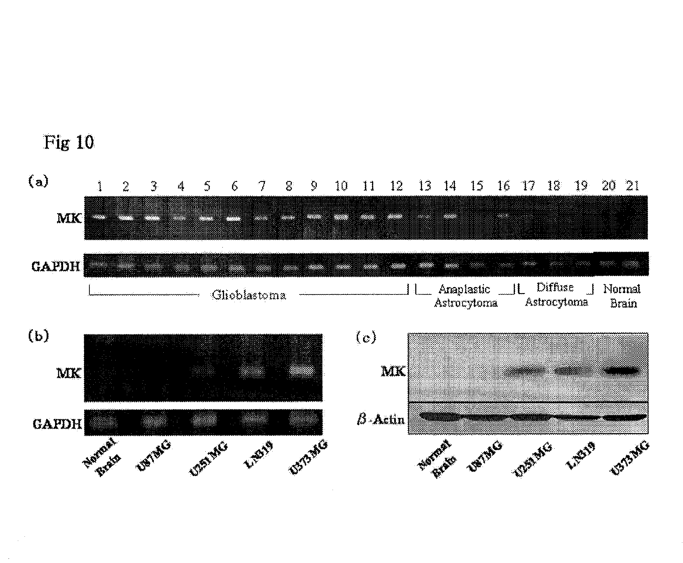

[Example 4: Antitumor effect by the use of a midkine promoter]
Then, antitumor effect by the use of a midkine promoter was investigated. Fig. 10 (a) shows the results of investigation of midkine (MK) mRNA expressions in human surgical samples 1-21 by RT-PCR. As shown by the Fig., excessive expression of the midkine mRNA was observed in malignant gliomas such as glioblastoma and anaplastic astrocytoma, and in diffuse astrocytoma. Thus, the excessive expression of midkine is observed in many cancers such as cerebral tumor.

Fig. 10 (b) shows the results of investigation of midkine mRNA expression by RT-PCR in four cell lines of malignant gliomas in a similar manner shown above. As shown by the Fig., no expression was observed in U87MG and excessive expression of the midkine mRNA were observed in U251MG, LN319 and U373MG.

Fig. 10 (c) shows the results of investigation of midkine protein expression in above mentioned each cell line by Western blotting analysis. No expression was found in U87MG as well as mRNA. Excessive expression of the midkine protein was observed in U251MG, LN319 and U373MG.

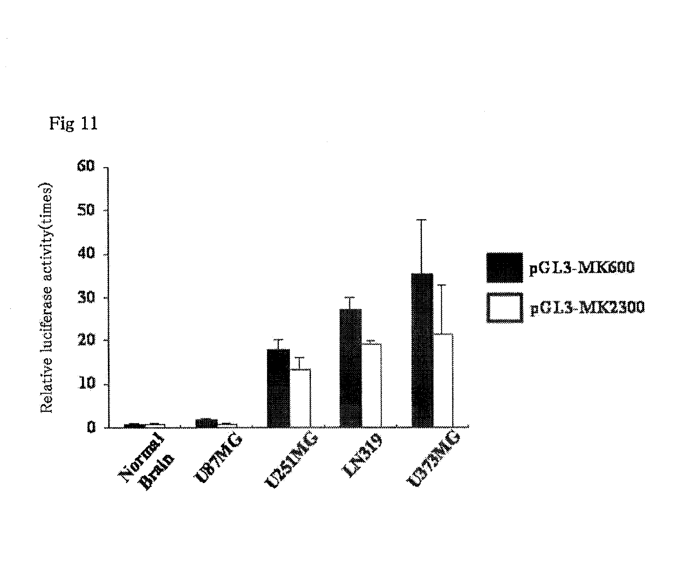

Then, a promoter assay of the midkine was performed. In the experiment, activity of two different length midkine promoters (600 bases and 2,300 bases) was compared. Plasmids (pGL3-MK600 and pGL3-MK2300) with inserted a luciferase gene at the downstream of respective promoters were introduced to each above mentioned cell line and their respective luciferase activity was investigated to evaluate the promoter activity. The results shown in Fig. 11 revealed a higher promoter activity in 600 base sequence length than that in 2,300 base sequence length in the malignant glioma cell line.

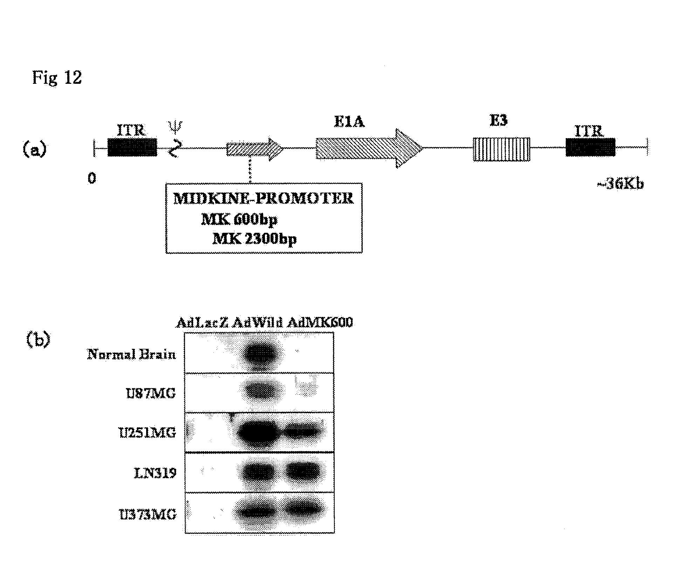

Fig. 12 (a) shows a schematic diagram of the oncolytic (cytolysis type) adenovirus structure having a midkine promoter designed in the present experiment. The midkine promoter having a 600 base sequence or a 2,300 base sequence was introduced at the site of 552 bp.

Fig. 12 (b) shows the results of investigation of E1A protein expression in above mentioned each cell line infected with three types of adenoviruses by Western blotting analysis. As shown in the Figure, expression of E1A protein of adenovirus was observed in midkine expressing U251MG, LN319 and U373, by the infection of adenovirus (AdMK600) having a 600 base length midkine promoter. Expression of E1A protein was observed in all cells including normal brain cells by wild type adenovirus (AdWild) and no expression of the E1A protein was observed in all cells by control virus AdLacZ.

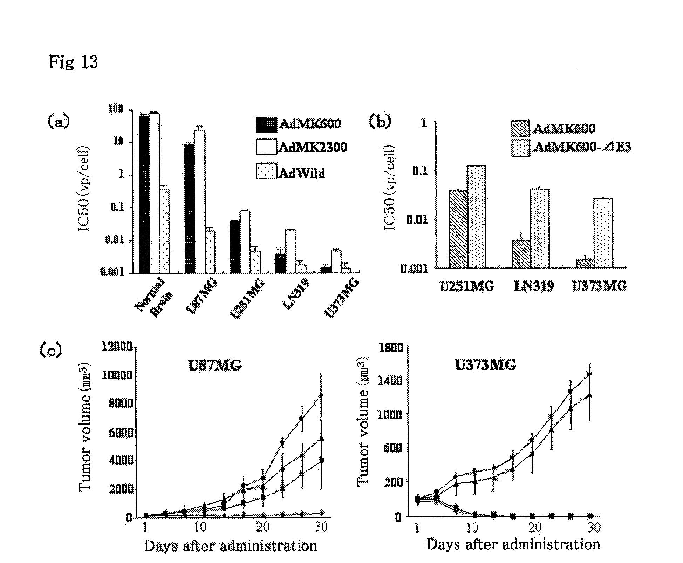

Fig. 13 (a) shows the results of comparative investigation of cancer cell proliferation inhibitory effect by three kinds of adenoviruses. Wild type adenovirus (AdWild) showed potent proliferation inhibitory effect in all cells, whereas adenoviruses (AdMK600 and AdMK2300) having the midkine promoter showed the proliferation inhibitory effect only in midkine expressing U251MG, LN319 and U373MG. These results were well correlated with the results of midkine mRNA expression and promoter activity. The adenovirus AdMK600 showed more potent proliferation inhibitory effect than that of AdMK2300 having 2,300 base sequence length.

Fig. 13 (b) shows the results of investigation of adenovirus E3 domain's influence on the proliferation inhibitory effect. As shown in the Figure, AdMK600 having E3 domain exhibited about 10-fold potent proliferation inhibitory effect than that of adenovirus having no E3 domain (AdMK600-ΔE3).

Fig. 13 (c) shows the results of investigation of antitumor effect of adenovirus in a nude mouse subcutaneous transplantation model of about 5-100 mm diameter tumor. In the Fig., black rhombus marks represent the results of administration of wild type adenovirus AdWild, black square marks represent the results of administration of adenovirus AdMK600 having a midkine promoter, black triangle marks represent the results of administration of adenovirus Ad-β-gal with inserted LacZ gene, and black round marks represent the results of administration of only PBS buffer. As shown in the Figure, only wild type adenovirus showed antitumor effect in the U87MG without midkine expression. In the U373MG expressing midkine, AdMK600 as well as AdWild gave complete disappearance of tumor. No marked difference was observed between the control with injected only PBS buffer and that with injected AdLacZ.

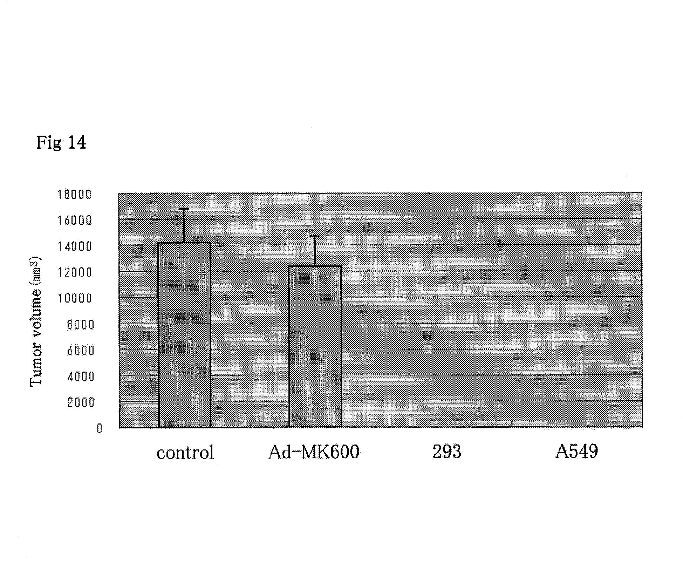

Furthermore, adenovirus having the above mentioned midkine promoter (Ad-MK600) was infected to the carrier cells and the antitumor effect of the resultant cancer gene therapeutic drug of the present invention was compared to that of administration of only Ad-MK600. In the experiment, 293 cells and A549 cells were used as carrier cells. Above mentioned U373MG cells were transplanted to 5-week-old nude mouse to give a 10-15 mm massive tumor after three weeks. The cancer gene therapeutic drug of the present invention or only Ad-MK600 was administered and the tumor volume was compared after four weeks. The results are shown in Fig. 14. In the Fig., "Ad-MK600" shows the results of administration of only Ad-MK600, and each "293" and "A549" show the results of administration of the cancer gene therapeutic drug of the present invention using 293 cells and A549 cells as carrier cells, respectively. As shown in the Figure, administration of the cancer gene therapeutic drug of the present invention showed complete disappearance of the tumor. Administration of only Ad-MK600 showed almost no difference with that of control.

Practically, favorable therapeutic effects on the ovarian cancer and malignant glioma were observed by application of carrier cells such as A549 cells and 293 cells and adenovirus having 1.A1.3B promoter or midkine promoter as the oncolytic virus. The midkine promoter can be used for various malignant tumors in addition to malignant glioma and is considered effective in the cancer therapy of other than malignant glioma.

[Example 5: Influences of Fe and ALA on the proliferation inhibitory effect of adenovirus AdE3-1A1.3B]
Ovarian cancer cells HEY were cultured in 12-well plate at a rate of 10,000 cells/well and FeSO₄ was added at a concentration of 50µg/ml, 5µg/ml, 0.5µg/ml or 0µg/ml on the following day and the cytolysis type adenovirus AdE3-1A1.3B was added to all wells. The proliferation inhibitory effect of the adenovirus was evaluated by IC₅₀ after five days. The results are shown in Fig. 15. In the Fig., the vertical axis shows relative rate (vp/cell) of viruses at IC₅₀ in each condition. As shown in the Figure, administration of 50µg/ml of FeSO₄ and the adenovirus showed about 20-fold, and administration of 5µg/ml of FeSO₄ and the adenovirus showed about 8-fold proliferation inhibitory effect, respectively, to that of only adenovirus administration.

Next, the ovarian cancer cell line HEY was cultured in a 12-well plate at a rate of 10,000 cells/well and 5-aminolevulinic acid (ALA) was added at a concentration of 50µg/ml, 5µg/ml, 0.5µg/ml or 0µg/ml on the following day and the cytolysis type adenovirus AdE3-1A1.3B was added to all wells. The proliferation inhibitory effect of the adenovirus was evaluated by IC₅₀ after five days. The results are shown in Fig. 16. In the Fig., the vertical axis shows relative rate (vp/cell) of viruses at IC₅₀ in each condition. As shown in the Fig., administration of 50µg/ml of ALA and the adenovirus showed about 100-fold proliferation inhibitory effect to that of only adenovirus administration.

Furthermore, the ovarian cancer cell line HEY was cultured in a 12-well plate at a rate of 10,000 cells/well and FeSO₄ was added at a concentration of 50µg/ml, 5µg/ml, 0.5µg/ml or 0µg/ml on the following day. In addition, the cytolysis type adenovirus AdE3-1A1.3B and 50µg/ml of 5-aminolevulinic acid (ALA) were added to each well. Only the adenovirus was added to a control. The proliferation inhibitory effect of the adenovirus was evaluated by IC₅₀ after five days. The results are shown in Fig. 17. In the Figure, the vertical axis shows relative administration rate (vp/cell) of viruses at IC₅₀ in each condition. As shown in the Fig., concurrent administration of 50µg/ml of FeSO₄, 50µg/ml of ALA and the adenovirus showed about 1,000-fold proliferation inhibitory effect to that of only adenovirus administration. Concurrent administration of 5µg/ml of FeSO₄, 50µg/ml of ALA and the adenovirus showed about 700-fold proliferation inhibitory effect to that of only adenovirus administration, and concurrent administration of 0.5µg/ml of FeSO₄, 50µg/ml of ALA and the adenovirus showed about 200-fold proliferation inhibitory effect to that of only adenovirus administration.

As mentioned above, it was found that ALA and Fe markedly enhance the proliferation inhibitory effect of the oncolytic adenovirus AdE3-1A1.3B. ALA and Fe elevate the infectivity of adenovirus and the amount of virus production, because β-gal assay revealed increased infectivity of adenovirus and PFU assay revealed increased amount of adenovirus production. That is, ALA and Fe can enhance antitumor effect, because they can increase the infectivity of adenovirus to cancer cells and the amount of virus production within the cells.

ALA is known to be a porphyrin metabolite taken up into cancer cells and its metabolite protoporphyrin IX is likely to be accumulated by the porphyrin metabolism. This compound has photo-sensitizing effect and it can be utilized for the photodynamic therapy (PDT) of superficial cancer, together with excimer dye laser.

Above mentioned protoporphyrin IX binds with Fe to give a heme and forms heme proteins such as cytochrome in cells. The heme proteins are involved in respiratory system in intracellular mitochondria, ATP production and protein synthesis. Thus, the heme proteins are involved in protein synthesis, including production of the adenovirus if the adenovirus infected. Therefore, promotion of the porphyrin metabolism may lead to the increased adenovirus production.

The cancer gene therapeutic drug of the present invention, as well as the cancer gene therapy of the present invention, can further increase the therapeutic effect by concurrent use of Fe and/or porphyrin compounds such as ALA. That is, concurrent use of Fe and/or porphyrin compounds such as ALA enhances antitumor effect, even under an infection suppressive condition in the presence of antibodies, by acceleration of the CTL response caused by the increased adenovirus production in the target cells. Concurrent use of Fe and/or porphyrin compounds can enhance antitumor effect not only in a syngenic mouse model with immune system but also in a human body.

In the cancer gene therapy using the oncolytic virus, concurrent use of Fe and/or porphyrin compounds such as ALA is expected to enhance the therapeutic effect, even if the carrier cells are not used.

[Example 6: Investigation for optimization of cancer therapy using the cancer gene therapeutic drug of the present invention]
The following a series of experiments was carried out to optimize cancer therapy using the cancer gene therapeutic drug of the present invention.

At first, an investigation of *in vivo* antitumor effect of the cancer gene therapeutic drug of the present invention was performed in a similar manner to the experiment shown in Fig. 7 using a subcutaneous tumor model mouse [(C57BL/6×C3/He) F1 mouse] with normal immune system. In the experiments, (C57BL/6×C3/He) F1 mouse of 5-week-old was immunized in advance by the administration of virus for immunological treatment, and twelve weeks later, ovarian cancer cells OVHM were subcutaneously transplanted at a rate of 1 ×10⁶ cells per mouse to form a 5-10 mm tumor. Then, A549 cells (Ad-A549) infected with the above mentioned adenovirus AdE3-1A1.3B at a rate of 250 vp/cell were administered in the tumor. A non-proliferative adenovirus having no E1. gene was used as the virus for immunological treatment, more specifically, adenovirus Ad-β-gal with inserted LacZ gene at the downstream of CMV promoter without inactivation by UV irradiation was used and intracutaneously administered at a rate of 1×10¹⁰ vp per mouse. The carrier cells, A549 cells, were irradiation treated at a dose of 200 Gy and were administered in the tumor of mouse at a rate of 5×10⁶ cells per once and six times in total.

The results of the above experiment are shown in Fig. 18 (a) and (b). In each graph, "Ad-β-gal→Ad-A549" represents the results of the above mentioned experiment, "Ad-A549" represents the results of administration of only the carrier cells, "Ad-β-gal" represents the results of administration of only the Ad-β-gal for treatment (not as the virus for immunological treatment), and "control" represents the results of administration of PBS buffer. Number (n) of mice in each group having five animals is shown by n=5. The graph (a) shows the observed results of tumor volume of each mouse for a comparatively short period and graph (b) shows the observed results of survival rate of mouse in each group for a long period. As shown by these graphs, a potent *in vivo* antitumor effect was observed in "Ad-β-gal→Ad-A549".

Administration interval between administration of the adenovirus for immunological treatment and that of the carrier cell was investigated. This experiment was similarly carried out as that in Fig. 18 except for the various changes of the administration intervals and infection of adenovirus AdE3-1A1.3B with carrier A549 cell at 50 vp/cell.

The results of the above mentioned experiments are shown in Fig. 19 (a) and (b). In each graph, "2-4w", "5-9w", "10-15w" and "16-22w" represent the results of experiments with above mentioned administration intervals of 2-4 weeks, 5-9 weeks, 10-15 weeks and 16-22 weeks, respectively. Number (n) of mice in each group having five animals is shown by n=5. As shown by these Figures, the best antitumor effect was obtained when above mentioned administration interval was set at 10-15 weeks. As shown by the present experiments, when the adenovirus Ad-β-gal without inactivation is administered as the virus for immunological treatment, the CTL reaction by T cells is considered to become predominant at about 10-15 weeks after the administration, compared with suppression of infection due to neutralizing antibodies.

Above mentioned administration interval is preferably to be short in consideration of the clinical application. Then, whether the above mentioned administration interval can be made short by the use of inactivated adenovirus Ad-β-gal as the virus for immunological treatment was investigated. As shown in Fig. 20, it was found that when the inactivated adenovirus UV-Ad-β-gal by UV irradiation was used as the virus for immunological treatment, the above mentioned administration interval set at four weeks or three weeks shows favorable antitumor effect, that is, inactivation of the virus for immunological treatment can shorten the above mentioned administration interval to about 3-4 weeks.

Above mentioned experiments were similarly carried out to those of experiments shown in Fig. 18 except for the following points; inactivated adenovirus UV-Ad-β-gal was used as the virus for immunological treatment, the above UV-Ad-β-gal was intracutaneously administered at a rate of 1×10⁷ vp per mouse, the above mentioned administration intervals were set to three weeks, four weeks, five weeks or six weeks, and carrier A549 cell was infected with the adenovirus AdB3-1A1.3B at a rate of 50 vp/cell. Number (n) of mice in each group having 10 animals is shown by n=10.

Fig. 21 shows the results of investigation of dosage of the virus when above mentioned UV-Ad-β-gal was used as the virus for immunological treatment. In this experiment, the rate of the UV-Ad-β-gal was changed in a range of 1×10⁶ vp to 1×10¹¹ vp. The experiment was carried out similarly with that shown in Fig. 20 except for above mentioned administration interval was set at six weeks. The result showed that the rate of UV-Ad-β-gal set at 1×10⁷ vp gave the best antitumor effect. (From this result, the rate of UV-Ad-β-gal was set at 1×10⁷ vp in the experiment shown in Fig. 20).

Fig. 22 (a) and (b) show the results of investigation of effect of tumor immunization (tumor vaccination). Above mentioned UV-Ad-β-gal was intracutaneously administered at a rate of 1×10⁷ vp per mouse and after 10 days, for tumor vaccination, irradiated ovarian cancer cells OVHM were subcutaneously transplanted at a rate of 1×10⁶ cells. Simultaneously, squamous ep. cancer cells SCC7 or ovarian cancer cells OVHM were subcutaneously transplanted at a rate of 1×10⁶ cells. Then, AdE3-1A1.3B infected A549 cells were administered at a rate of 5×10⁶ cells to mice six times in a formed 5-10 mm tumor (in the Fig., shown by "OVHM-RT+Ad-β-gal→SCC7" and "OVHM-RT+Ad-β-gal→OVHM"). The administered mouse showed marked inhibition of tumor growth and proliferation in comparison to the control group (in the Fig., "SCC7" represents SCC7 tumor treated by the carrier cell without tumor vaccination, and "OVHM" represents OVHM tumor treated by the carrier cell without tumor vaccination). Especially, by the tumor vaccination with irradiated OVHM followed by the carrier cell treatment, formed OVHM tumor completely disappeared in all mice without recurrence.

Fig. 23 shows the results of investigation of tumor vaccination with non-small-cell lung cancer A549 cells. In this experiment, irradiated A549 cells infected with adenovirus AdE3-1A1.3B at a rate of 1.00 vp/cell were subcutaneously transplanted at a rate of 1×10⁶ cells per mouse. After 40 days, ovarian cancer cells OVHM were subcutaneously transplanted at a rate of 1×10⁶ cells. The mice (in the Fig., "AdE3-1.3B-infected A549→OVHM") also showed marked inhibition of tumor growth and proliferation in comparison to the control group (in the Fig., "OVHM" represents OVHM tumor treated by the carrier cell without tumor vaccination) with marked improvement in survival rate.

Above mentioned results show that the antitumor effect can be obtained even by tumor vaccination with different kinds of cancer cells.

Then, the effect of administration of atelocollagen together with the carrier cell was investigated. In this experiment, A549 cells infected with a predetermined amount of adenovirus AdE3-1A1.3B were administered to 5- to 10-week-old (C57BL/6×C3/He) F1 mouse at a rate of 5×10⁶ cells by mixing atelocollagen to make final concentration of 0.1%, and it was investigated whether the death rate caused by the side effect due to the administration of adenovirus was improved. The results are shown in Fig. 24. In the Fig., a right bar represents the results of administration of atelocollagen together with adenovirus AdE3-1.3B infected A549 cells at a rate of 50 vp/cell or 250 vp/cell. Left and central bars represent the results of administration of adenovirus AdE3-1.3B infected A549 cells at a rate of 5 vp/cell and 50 vp/cell, respectively (no atelocollagen is mixed). As shown in the Fig., simultaneous administration of atelocollagen dramatically reduced the death rate caused by the side effect and the administration dose can be increased. This may be caused by the inhibition of adenovirus dispersion and the blockage of anti-adenovirus neutralizing antibody by the atelocollagen.

As shown above, simultaneous administration of atelocollagen and carrier cells suppressed the side effect and a high dose administration of adenovirus became possible.
Next, the adenovirus Ad-β-gal without inactivation treatment was administered once, twice or thrice into a mouse, to increase anti-adenovirus antibody in the blood by the booster effect. After that, antitumor effect was investigated in each mouse. In this experiment, adenovirus Ad-β-gal was once, twice or thrice administered to 5-week-old (C57BL/6×C3/He) F1 mouse (administration at every four weeks at a rate of 1×10¹⁰ vp), thereafter, ovarian cancer cells OVHM were subcutaneously transplanted at a rate of 1×10⁶ cells per mouse to form 5-10 mm tumor and irradiated carrier cells were administered in the tumor. A549 cells, optionally mixed with 293 cells, were used as carrier cells.

In case of mixtures of A549 cells and 293 cells, A549 cells infected with adenovirus AdE3-1A1.3B at a rate of 50 vp/cell were administered at a rate of 3.75×10⁶ cells, and 293 cells infected with adenovirus AdE3-1A1.3B at a rate of 10 vp/cell were administered at a rate of 3.75×10⁶ cells, respectively, in the tumor for one administration. These two kinds of carrier cells were administered six times in total. The results are shown in Fig. 25 (a) and (b). In case of administration of only A549 cells, A549 cells infected with adenovirus AdE3-1A1.3B at a rate of 50 vp/cell were administered at a rate of 7.5×10⁶ cells per mouse in the tumor for one administration. The carrier cells were administered six times in total. The results are shown in Fig. 26 (a) and (b). In the Fig. 25 and Fig. 26, "×1" "×2" and "×3" represent the results of once, twice and thrice administrations of adenovirus Ad-β-gal, respectively. As shown in these figures, carrier cells showed antitumor effect even in anti-adenovirus antibody positive mouse, caused by the several administrations of adenovirus Ad-β-gal. The mixture of A549 cells and 293 cells used as carrier cells showed superior result to that of administration of only A549 cells.

Fig. 27 (a) and (b) show the results of investigation of *in vivo* antitumor effect by administration of carrier cells (A549 cells) infected with not only adenovirus AdE3-1A1.3B but also GM-CSF expression vector, and further together with atelocollagen. In this experiment, adenovirus Ad-13-gal was administered once, twice or thrice (every four weeks at a rate of 1×10¹⁰ vp) to 5-week-old (C57BL/6×C3/H3) F1 mouse in a similar manner with experiment shown in Fig. 26.
Then, ovarian cancer cells OVHM were subcutaneously transplanted at a rate of 1×10¹⁰ cells per mouse. After formation of tumor with 5-10 mm diameter, irradiated carrier cells (A549 cells) were administered in the tumor. The carrier cells were infected with adenovirus AdE3-1A1.3B at a rate of 50 vp/cell and a GM-CSF expression vector (a vector with inserted a GM-CSF gene at the adenovirus E1 gene deficient site and at the downstream of CMV promoter) at a rate of 10 vp/cell. The prepared irradiated A549 cells were administered in the tumor at a rate of 7.5×10⁶ cells, together with atelocollagen (concentration at 0.1.%), for one administration. These were three times (x3) administered in total.

In Fig. 27 (a) and (b), "Ad-β-gal→AdE3-1A1.38+GMCSF" represents the results of above mentioned experiment, "Ad-β-gal→AdE3-1A1.3B" represents the results of six times (x6) administrations of carrier cells (A549 cells) infected with adenovirus AdE3-1A1.3B at a rate of 50 vp/cell in the tumor at a rate of 7.5×10⁶ cells for one administration. As shown in the Figure, three times administrations of "AdE3-1A1.3B+GMCSF" showed more potent *in vivo* antitumor effect than that of six times administrations of "AdE3-1A1.3B", in all once, twice and thrice administrations of adenovirus Ad-β-gal.

Above results showed that infection of not only the oncolytic adenovirus but also GM-CSF expression vector to the carrier cells is very effective in cancer therapy.

Fig. 28 (a) and (b) show the results of investigation of effect of intraperitoneal administration of an iron preparation at the time of carrier cell administration. In this experiment, adenovirus Ad-β-gal was once, twice or thrice administered (every four weeks at a rate of 1×10¹⁰ vp for one administration) to 5-week-old (C57BL/6×C3/He) F1 mouse. Then ovarian cancer cells OVHM were subcutaneously transplanted at a rate of 1×10⁶ cells per mouse. After formation of tumor with 5-10 mm diameter, irradiated carrier cells were administered in the tumor. A549 cells infected with adenovirus AdE3-1A1.3B at a rate of 50 vp/cell were used as the carrier cells and administered at a rate of 7.5×10⁶ cells for one administration. At the time of carrier cell administration, 0.01 mg of iron dextran (Fe-Dextran) was intraperitoneally administered as an iron preparation. These were three times (x3) administered in total (the iron preparation was also administered in every occasion).

In Fig. 28 (a) and (b), "Ad-β-gal→AdE3-1A1.3B+Fe" represents the results of above experiment. "Ad-β**-**gal→AdE3-1A1.3B" represents the results of six times (x6) administrations of carrier cells (A549 cells) infected with adenovirus AdE3-1A1.3B at a rate of 50 vp/cell in the tumor at a rate of 7.5×10⁶ cells for one administration. As shown in the Figure, three times administrations of "AdE3-1A1.3B+Fe" showed more potent *in vivo* antitumor effect than that of six times administrations of "AdE3-1A1.3B" (in which case, only the carrier cells was administered), in all once, twice and thrice administrations of adenovirus Ad-β-gal.

Above results showed that a combined administration of the carrier cell with an iron preparation is very effective in cancer therapy.

Then, a radiation dose in the radiation exposure treatment to the carrier cells before administration was investigated. In the experiment, 5-week-old nude mouse was used and A549 cells were irradiated at different doses and then subcutaneously transplanted at a rate of 1×10⁷ cells per mouse, and the formation and growth of the tumor was observed. The results are shown in Fig. 29. As shown in the Figure, formation and growth of tumor was inhibited by setting the radiation dose at 120 Gy or over.

In an experiment using (C57BL/6×C3/He) F1 mouse, Fig. 30 shows the results of investigation of antitumor effect when the carrier cells (A549 cells) were treated by radiation with various doses. In this experiment, adenovirus UV-Ad-β-gal was administered to 5-week-old (C57BL/6×C31He) F1 mouse at a rate of 1×10¹⁰ vp. After five weeks, the ovarian cancer cells OVHM were subcutaneously transplanted at a rate of 1×10⁶ per mouse. After formation of tumor with 5-10 mm diameter, carrier cells irradiated at a dose of 50 Gy, 100 Gy, 200 Gy or 400 Gy were administered in the tumor. A549 cells infected with adenovirus AdE3-1A1.3B at a rate of 50 vp/cell were used as the carrier cells and administered at a rate of 7.5×10⁶ cells for one administration. The carrier cells were six times administered in total. The results showed a more favorable result at radiation dose of 400 Gy than that at radiation dose of 200 Gy.

Fig. 31 shows the results of investigation for the amount of infection of the oncolytic virus to the carrier cell. In this experiment, adenovirus Ad-β-gal was administered to 5-week-old (C57L/6×C3/He) F1 mouse at a rate of 1×10¹⁰ vp. After four weeks, ovarian cancer cells OVHM were subcutaneously transplanted at a rate of 1×1⁶ cells. After formation of tumor with 5-10 mm diameter, carrier cells (A549 cells) irradiated at a dose of 250 Gy were administered in the tumor. The amount of infection of adenovirus AdE3-1A1.3B to the carrier cell was set 100 vp/cell, 250 vp/cell or 500 vp/cell. The carrier cells were administered at a rate of 7.5×10⁶ cells for one administration. Together with the carrier cell administration, atelocollagen (concentration 0.1%) was concurrently administered in the tumor. These were six times administered in total. The results showed most favorable result at the infection rate of 250 vp/cell, and found that rates of 150-400 vp/cell gave favorable results.

Fig. 32 (a) and (b) show the results of investigation of effect of tumor vaccination in a similar experiment to that shown in Fig. 31. In this experiment, adenovirus Ad-β-gal was administered to 5-week-old (C57L/6×C3/He) F1 mouse at a rate of 1×10¹⁰ vp. After four weeks, for tumor vaccination, ovarian cancer cells OVHM-RT irradiated at a dose of 80 Gy was subcutaneously transplanted at a rate of 1×10⁶ cells. Then, ovarian cancer cells OVHM were subcutaneously transplanted at a rate of 1×10⁶ cells. After formation of tumor with 5-10 mm diameter, carrier cells treated irradiated at a dose of 250 Gy was administered in the tumor. A549 cells infected with adenovirus AdE3-1A1.3B at a rate of 50 vp/cell were used as the carrier cells and administered at a rate of 7.5×10⁶ cells for one administration. Together with the carrier cell administration, atelocollagen (concentration 0.1%) was concurrently administered in the tumor. These were three times administered in total. The results showed marked inhibition of tumor growth and proliferation in the mouse with tumor vaccination (in the Fig., "OVHM-RT→A549") in comparison to the mouse without tumor vaccination (in the Fig., "A549"). The survival rate was also greatly improved.

Above results show that the use of tumor vaccination provides favorable antitumor effect together with the use of the cancer gene therapeutic drug of the present invention.

### Industrial applicability

As described above, the cancer gene therapeutic drug of the present invention can be applied almost all malignant tumors and can be expected to exhibit potent antitumor effect including ovarian cancer, squamous epithelium cancers (e.g. uterine cervix cancer, cutaneous carcinoma, head and neck cancer, esophageal cancer and lung cancer), digestive tract cancers (e.g. colonic cancer, pancreatic cancer, hepatic cancer and gastric cancer), neuroblastoma, cerebral tumor, mammary cancer, testicular cancer and prostatic cancer.

## Claims

1. A cancer gene therapeutic drug including a carrier cell to be infected with an oncolytic virus, so as to make the oncolytic virus act on a tumor cell within a living body, wherein the carrier cell is selected from the following (1) to (4) cells:
(1) A549 cell
(2) 293 cell
(3) SW626 cell, and
(4) HT-3 cell.

2. The cancer gene therapeutic drug according to claim 1, wherein the oncolytic virus to be infected to the carrier cell has 1A1.3B promoter, midkine promoter, β-HCG promoter, SCCA1 promoter, cox-2 promoter, PSA promoter or another tumor specific promoter, according to a kind of cancer to be treated etc.

3. The cancer gene therapeutic drug according to claim 1 or 2, wherein the oncolytic virus is selected from adenovirus, herpes virus, lentivirus such as HIV virus, retrovirus, reovirus, vesicular stomatitis virus (VSV) and any other oncolytic viruses.

4. The cancer gene therapeutic drug according to claim 1, further comprising a virus for immunological treatment to be administered for inducing a CTL reaction within the living body to administration of the carrier cell.

5. The cancer gene therapeutic drug according to claim 1, further comprising atelocollagen.

6. The cancer gene therapeutic drug according to claim 1, further comprising a GM-CSF expression vector to be infected to the carrier cell before administration.

7. The cancer gene therapeutic drug according to claim 1, further comprising an iron preparation and/or a porphyrin compound.

8. The cancer gene therapeutic drug according to claim 1, further comprising a tumor cell to be administered for tumor vaccination.
